# EUROPÄISCHE PATENTSCHRIFT

(11) **EP 0 513 782 B1**
(45) Veröffentlichungstag und Bekanntmachung des Hinweises auf die Patenterteilung: **22.03.1995**
(21) Anmeldenummer: 92108146.9
(22) Anmeldetag: 14.05.1992
(51) Int. Cl.: C07C 233/36, C07C 233/38, C07C 233/40, C09F 1/04, B01F 17/52

(54) **Stickstoffhaltige, grenzflächenaktive Mittel, abgeleitet aus Harzsäuren**
Nitrogen containing surface active agents derived from rosin acids
Agents surfactants, qui contiennent la nitrogen et qui sont dérivés des acides résiniques

(30) Priorität: 17.05.1991 DE 4116111
(43) Veröffentlichungstag der Anmeldung: 19.11.1992
(73) Patentinhaber: HOECHST AKTIENGESELLSCHAFT, 65926 Frankfurt am Main (DE)
(72) Erfinder: Uhrig, Heinz, W-6374 Steinbach/Taunus (DE); Schwerin, Siegfried, Dr., W-6238 Hofheim/Taunus (DE); Schnaitmann, Dieter, Dr., W-6239 Eppstein/Ts. (DE); Metz, Hans Joachim, Dr., W-6100 Darmstadt (DE)

(56) Entgegenhaltungen:
- EP-A- 0 009 648
- EP-A- 0 296 543
- EP-A- 0 341 593

## Beschreibung

Die Erfindung betrifft neue oberflächenaktive Substanzen, ein Verfahren zu deren Herstellung sowie deren Verwendung.

Bei der Herstellung von Präparationen von Farbmitteln, insbesondere von Pigmenten, für die Anwendung in wäßrigen und organischen Medien werden häufig nichtionogene, anionische und auch kationische Tenside verwendet. Solche Tenside haben wegen ihrer oberflächenaktiven Eigenschaften einen großen Einfluß auf die coloristischen Eigenschaften der Farbmittel in den verschiedenen Anwendungsmedien, besonders auf dem Drucksektor. Tenside werden üblicherweise auch zum besseren Ablauf der Kupplungsreaktion bei der Herstellung von Azopigmenten und löslichen Azofarbstoffen sowie zur Präparierung der Farbmittel benutzt. Die ständig steigenden Anforderungen an die coloristischen und rheologischen Eigenschatten der Azopigmente machen besonders auf dem Gebiet der Druckfarben eine gezielte Entwicklung von Präparationsmitteln notwendig, die die Fließfähigkeit von Druckfarben verbessern.

Bei der Herstellung von Druckfarben werden beispielsweise Veresterungsprodukte von Harzsäuren und modifizierten Harzsäuren mit Polyalkoholen, beispielsweise Glycerin, Pentaerythrit und Sorbit verwendet (Ullmanns Encyklopädie der technischen Chemie, 3. Aufl., Band 8, Seite 412, und J. Scheiber, Chemie und Technologie der künstlichen Harze, 5. Auflage, 1943, Seite 560).

Aus den US-PS 4,297,270 und 4,312,631 sind sulfosuccinatgruppenhaltige Oxalkylate auf Basis von Harzsäureestern von Polyalkoholen als Dispergiermittel für die Feinverteilung und Stabilisierung von Feststoffen sowie als Netz-, Emulgier-, Egalisier- und Färbereihilfsmittel bekannt.

In den US-PS 3,841,888 und 3,947,287 werden wäßrige, flockungsstabile Pigmentdispersionen beschrieben, die unter Zuhilfenahme von Blockpolymerisaten aus oxalkylierten aliphatischen, aromatischen oder cycloaliphatischen Diaminen zubereitet sind.

In der US-PS 4,769,759 werden für das Färben von Wolle mit anionischen Farbstoffen Färbereihilfsmittel beschrieben, die man durch Halbveresterung von oxalkylierten Fettaminen mit Maleinsäure- oder Phthalsäureanhydrid erhält.

Weiterhin werden in der japanischen Patentbekanntmachung Sho-59-071486 Blockpolymerisate von Ethylendiaminderivaten mit einem Alkylenoxid als Hilfsmittel beim Klotzen von Dispersionsfarbstoffen auf hydrophoben Synthesefasern erläutert. Schließlich sind aus der japanischen Patentbekanntmachung Sho-60-024 229 mit Styrol modifizierte, teils mit Schwefelsäure sulfatierte Blockpolymerisate von oxalkylierten Alkylendiaminen für den Einsatz als Egalisiermittel beim Färben von synthetischen Materialien bekannt.

Keines der in den vorstehend genannten Druckschriften beschriebenen Produkte ist jedoch geeignet, die Fließfähigkeit von Druckfarben entscheidend zu verbessern, ohne andere Parameter wie Farbstärke, Glanz, Farbton und Dispergierbarkeit nachteilig zu beeinflussen.

Der vorliegenden Erfindung liegt die Aufgabe zugrunde, grenzflächenaktive Mittel zur Verfügung zu stellen, die zur Herstellung gut fließfähiger Feststoffdispersionen, vorzugsweise Farbmittelpräparationen für den Offsetdruck, geeignet und weitgehend frei von den obengenannten Nachteilen sind.

Durch Veresterung von Oxalkylaten auf Basis von modifizierten oder unmodifizierten natürlichen Harzsäuren oder daraus abgeleiteten Harzaminen oder Harzalkoholen mit Dicarbonsäuren oder deren Anhydriden und anschließender Umsetzung mit Alkylendiaminen oder Alkylenpolyaminen werden schwach gelbliche, schaumarme grenzflächenaktive Substanzen erhalten, die überraschenderweise zur Herstellung gut fließfähiger Feststoffdispersionen, insbesondere gut fließfähiger Farbmittelpräparationen geeignet sind.

Gegenstand der vorliegenden Erfindung sind nun Mischungen enthaltend im wesentlichen Verbindungen der Formel (I)

A[(B)ₘ-Y-Z]_{q} (I)

in der
- A: für den Rest einer modifizierten oder unmodifizierten natürlichen Harzsäure oder eines daraus abgeleiteten Harzamins oder Harzalkohols oder für den Rest eines Veresterungsprodukts von 1 bis 6, vorzugsweise 1 bis 2, Einheiten der genannten Harzsäuren mit einem mehrwertigen Alkohol oder eines Veresterungsprodukts von 1 bis 12, vorzugsweise 1 bis 6, Einheiten der genannten Harzsäuren mit einem Aminooxalkylat aus 1 bis 5, vorzugsweise 1 bis 3, Einheiten der Verbindung nach der Formel (II) steht, 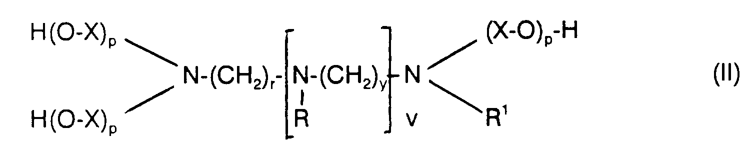
wobei das Veresterungsprodukt noch mindestens eine freie Hydroxygruppe enthält,
worin
- X: für eine Gruppe der Formeln -CH₂CH₂-, -CH₂CH(CH₃)- oder -CH(CH₃)CH₂- oder für eine Kombination daraus;
- R: für ein Wasserstoffatom oder die Gruppe -(X-O)ₚ-H
- R¹: für die Gruppe -(X-O)ₚ-H oder die Gruppe -(X-O)ₚ-R², worin R² für eine divalente Gruppe -OC-E-CO- steht, welche über die gezeigten freien Valenzen zwei Einheiten der Verbindung der Formel (II) esterartig verknüpft, worin E für einen divalenten aromatischen Rest mit 6 bis 12 C-Atomen oder für eine geradkettige, verzweigte oder cycloaliphatische Alkylengruppe mit 1 bis 16 C-Atomen, vorzugsweise eine geradkettige oder verzweigte Alkylengruppe mit 1 bis 8 C-Atomen, v für eine ganze Zahl von Null bis 4, vorzugsweise von Null bis 2, und r und y gleich oder verschieden sind und jeweils für eine ganze Zahl von 1 bis 5 stehen;
- p: eine ganze Zahl von 1 bis 100, vorzugsweise von 1 bis 20, bedeutet
- B: eine direkte Bindung, wenn A für ein Veresterungsprodukt aus mindestens einer der obengenannten Harzverbindungen mit mindestens einem Rest einer Verbindung der Formel (II) steht,
oder eine Gruppe der Formel -(X-O)- bedeutet, worin X die obengenannte Bedeutung hat,
- Y: für eine Gruppe der Formeln -OC-F-CO- und -OC-F-COO⁻ steht, worin F für einen divalenten aromatischen Rest mit 6 bis 12 C-Atomen oder für eine geradkettige, verzweigte oder cycloaliphatische Alkylengruppe mit jeweils 1 bis 16 C-Atomen, vorzugsweise für geradkettiges oder verzweigtes C₁-C₈-Alkylen, steht und
- Z: für eine Gruppe der Formel (IIIa) 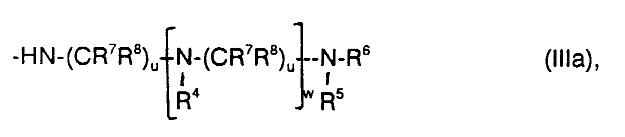 wenn Y die Bedeutung -OC-F-CO- hat
oder für ein Kation der Formel (IIIb) steht 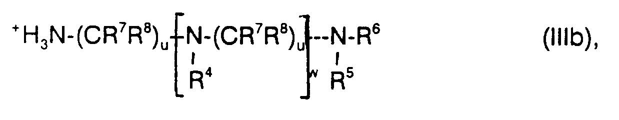 wenn Y die Bedeutung -OC-F-COO⁻ hat,
worin R⁴, R⁵ und R⁶ unabhängig voneinander für ein Wasserstoffatom oder ein Hydroxyalkylen mit 1 bis 6 C-Atomen, vorzugsweise mit 2 bis 3 C-Atomen, R⁷ und R⁸ unabhängig voneinander für Wasserstoff oder Methyl, u gleich oder verschieden ist und für eine ganze Zahl von 1 bis 14, bevorzugt von 2 bis 3, w für eine ganze Zahl von Null bis 25, bevorzugt von Null bis 5, stehen und
- m: eine Zahl von 1 bis 100, vorzugsweise 5 bis 30, und
- q: eine ganze Zahl von 1 bis 11, vorzugsweise 1 bis 6, bedeutet.

Unter "Rest" wird jeweils die Stammverbindung abzüglich mindestens eines Wasserstoffatoms verstanden.

Von besonderem Interesse sind Verbindungen der Formel (I), in der A ein Rest einer natürlichen Harzsäure, einer hydrierten oder disproportionierten Harzsäure, eines daraus abgeleiteten Harzamins oder Harzalkohols oder den Rest eines Veresterungsprodukts bedeutet, das durch Veresterung von 1 bis 6, vorzugsweise 1 bis 2, Einheiten der vorstehenden Harzsäuren mit einem 2- bis 6-wertigen Alkohol oder von 1 bis 12, vorzugsweise 1 bis 6, Einheiten der vorstehenden Harzsäuren mit 1 bis 5, vorzugsweise 1 bis 3, Einheiten der Formel (II) enthaltenden Aminooxalkylat unter Beibehaltung von mindestens einer freien Hydroxygruppe erhalten wird.

Von besonderem Interesse sind ferner Verbindungen der Formel (I), in der B eine direkte Bindung bedeutet, wenn A für ein Veresterungsprodukt, hergestellt aus mindestens einer der vorstehend genannten Harzverbindungen mit einem Aminooxalkylat aus 1 bis 5, vorzugsweise 1 bis 3, Einheiten der Verbindung gemäß Formel (II), steht.

Von besonderem Interesse sind weiterhin Verbindungen der Formel (I), in der B die Gruppe der Formel -(X-O)- bedeutet, worin X für eine Gruppe der Formeln -CH₂CH₂-, -CH₂CH(CH₃)- und -CH(CH₃)-CH₂- oder für eine Kombination daraus steht.

Von besonderem Interesse sind außerdem Verbindungen der Formel (I), in der Y eine Gruppe der Formeln -OC-F-CO- oder -OC-F-COO⁻ bedeutet, worin F vorzugsweise für eine geradkettige oder verzweigte Alkylengruppe mit jeweils 1 bis 8 C-Atomen, insbesondere 1 bis 4 C-Atomen, oder für einen divalenten aromatischen Rest mit 6 bis 8 C-Atomen steht.

Von Interesse sind auch Verbindungen der Formel I, in der Z für eine Gruppe der Formeln (IIIa) und (IIIb) steht, worin R⁴ und R⁵ jeweils ein Wasserstoffatom, R⁶ ein Wasserstoffatom oder ein Hydroxyalkylen mit 2 bis 3 C-Atomen sind und u und w die vorn genannten Bedeutungen haben.

Von besonderem Interesse sind ebenfalls Verbindungen der Formel (I), in der Z für eine Gruppe der Formeln (IIIa) und (IIIb) steht, worin R⁴, R⁵ und R⁶ jeweils ein Wasserstoffatom, u gleich oder verschieden ist und die Zahl 2 oder 3 und w eine ganze Zahl von Null bis 5 bedeuten.

Gegenstand der Erfindung ist auch ein Verfahren zur Herstellung der erfindungsgemäßen Verbindungen. Sie werden erhalten, indem man
a1) natürliche Harzsäuren, davon abgeleitete Harzalkohole oder Harzamine, disproportionierte oder hydrierte Harzsäuren oder Veresterungsprodukte der genannten Harzsäuren mit mehrwertigen Alkoholen, wobei die Veresterungsprodukte noch mindestens eine freie Hydroxygruppe enthalten, mit Ethylenoxid oder Propylenoxid oder beiden Epoxiden hintereinander oder einem Gemisch beider Epoxide oxalkyliert, wobei pro reaktionsfähigem Wasserstoffatom der verwendeten Harzverbindung 1 bis 100 Mol an Epoxid eingesetzt werden,
oder a2) Oxalkylierungsprodukte, die man durch Veresterung der genannten Harzsäuren mit Aminooxalkylaten der Formel (II) erhält,
b) mit Dicarbonsäuren oder Dicarbonsäureanhydriden an den endständigen Hydroxygruppen halbverestert
c) und anschließend die freien Carboxylgruppen der überwiegend entstandenen Carbonsäureverbindung A[(B)ₘ-CO-F-COOH]_{q} mit mindestens einem der Formel Z zugrundeliegenden Diamin oder Polyamin in die jeweilige Amidform oder Salzform überführt.

Bei der Herstellung der erfindungsgemäßen Verbindungen wird, bedingt durch die Vielzahl der Reaktionszentren in den Ausgangsverbindungen, kein reines, einheitliches Endprodukt erhalten, sondern eine Mischung, die jedoch Verbindungen gemäß der Formel (I) als Hauptkomponente, das heißt zu mehr als 50 Gew.-%, vorzugsweise zu mehr als 90 Gew.-%, enthält.

Der Umsetzungsgrad in den oben beschriebenen Reaktionsstufen a) und b) wird in den Herstellungsbeispielen durch die Hydroxylzahl und die Säurezahl charakterisiert und der Umsetzungsgrad zum Endprodukt durch die Aminzahl.

Die Molmasse der erfindungsgemäßen Verbindungen kann in einem weiten Bereich schwanken und reicht von 800 bis 50000, bevorzugt 1000 bis 10000, insbesondere 1000 bis 3000.

Folgende dem Rest A zugrundeliegenden Harzverbindungen eignen sich beispielsweise zur Herstellung der erfindungsgemäßen Verbindungen:
a) natürliche Harzsäuren und ihre Hydrierungs- oder Disproportionierungsprodukte, wobei die genannten Harzverbindungen vorzugsweise in Form der handelsüblichen Kolophoniumarten vorliegen oder daraus gewonnen werden;
b) Harzamine, wie sie aus den unter a) genannten Harzsäuren durch Umwandlung zum Harzsäurenitril und anschließender Hydrierung erhalten werden;
c) Harzalkohole, wie sie aus den unter a) genannten Harzsäuren durch Reduktion, insbesondere Hydrierung, entstehen;
d) Veresterungsprodukte, wie man sie durch Veresterung einer unmodifizierten oder einer modifizierten natürlichen Harzsäure, wie sie unter a) genannt werden, mit einem 2- bis 6-wertigen Alkohol oder einem Aminooxalkylat der Formel (II) erhält, wobei im Veresterungsprodukt noch 1 bis 11, vorzugsweise 2 bis 6, freie Hydroxygruppen vorhanden sein müssen.

Als Ausgangsprodukte eignen sich vorzugsweise natürliche Harzsäuren, wie Abietinsäure, Dehydroabietinsäure, Dihydroabietinsäure, Tetrahydroabietinsäure, Laevopimarsäure, Dextropimarsäure und Isodextropimarsäure, wie sie in handelsüblichen Kolophoniumarten vorliegen. Weiterhin eignen sich vorzugsweise disproportionierte, hydrierte und dimerisierte Harzsäuren, Harzalkohole, wie Abietylalkohol und Hydroabietylalkohol, wobei auch das handelsübliche Gemisch aus Dehydroabietyl-, Dihydroabietyl- und Tetrahydroabietylalkohol (technischer Hydroabietylalkohol) geeignet ist, sowie Harzamine, die von den obengenannten Harzsäuren abgeleitet sind, insbesondere Dehydroabietylamin.

Zur Veresterung der Harzsäuren bei der Herstellung der unter d) genannten Harzverbindungen sind beispielsweise folgende mehrwertige Alkohole geeignet: 1,2-Ethandiol, 1,2-Propandiol, 1,2- und 1,4-Butandiol, 1,2,4-Butantriol, 1,1,1-Trimethylolpropan, Glycerin, Polyglycerin, Pentaerythrit, Dipentaerythrit, Trimethylolethan, Neopentylglykol, 2,4-Dihydroxy-3-methylol-pentan, 1,2,6-Hexantriol, Sorbit, Anhydrosorbit, Hexit und Mannit, vorzugsweise Glycerin und Polyglycerin.

Zur Veresterung der Harzsäuren bei der Herstellung der unter d) genannten Harzverbindungen sind gleichermaßen die Aminooxalkylate der Formel (II) geeignet. Aminooxalkylate der Formel (II) sind in K. Lindner, Tenside, Textilhilfsmittel, Waschrohstoffe, Springer-Verlag, 1964, Seite 1055 bis 1056 beschrieben. Die Herstellung der Aminooxalkylate der Formel (II) kann nach üblichen Methoden erfolgen, indem man das entsprechende Diamin oder Polyamin bei einer Temperatur von 80 bis 160 °C, vorzugsweise 110 bis 140°C und einem Druck von 2 bis 8 bar, vorzugsweise 3 bis 5 bar, zweckmäßigerweise unter Zusatz von alkalischen Katalysatoren, mit Ethylenoxid, Propylenoxid oder einem Gemisch davon oxalkyliert. Die Verknüpfung von zwei oder mehreren Aminooxalkylateinheiten der Formel (II) kann durch Veresterung eines Aminooxalkylates in mindestens einer Reaktionsstufe mit mindestens einer Dicarbonsäure oder Dicarbonsäureanhydrid im Molverhältnis 2:1 bis 5:4, vorzugsweise 2:1 bis 3:2 von Aminooxalkylat zu Dicarbonsäure(anhydrid) durchgeführt werden.

Als Dicarbonsäuren sind zur Herstellung der erfindungsgemäßen Verbindungen vorzugsweise aliphatische Dicarbonsäuren mit 3 bis 12 Kohlenstoffatomen geeignet, insbesondere Malonsäure, Bernsteinsäure, Glutarsäure, Adipinsäure, 1,5-Pentandicarbonsäure, 1,6-Hexandicarbonsäure und 1,10-Decandicarbonsäure, aber auch beispielsweise Cyclohexan-1,4-dicarbonsäure sowie Phthalsäure und Terephthalsäure. Als Anhydride sind insbesondere Maleinsäureanhydrid, Bernsteinsäureanhydrid, Glutarsäureanhydrid und Phthalsäureanhydrid geeignet.

Die Verknüpfung der Aminooxalkylate mit den genannten Dicarbonsäuren oder deren Anhydriden kann nach an sich üblichen Veresterungsmethoden erfolgen. Die Reaktionstemperatur liegt dabei je nach Veresterungsmethode zwischen 0°C und 240°C, vorzugsweise 130 bis 180°C. Bevorzugt wird zur Erhöhung der Ausbeute die Veresterung in einem inerten organischen Lösungsmittel durchgeführt, das als Schleppmittel zum Entfernen des Reaktionswassers geeignet ist. Beispielsweise kann die Veresterung in Xylol als organischem Lösungsmittel und in Gegenwart von sauren Katalysatoren bei einer Temperatur von 130 bis 220°C, vorzugsweise 140 bis 170°C, durchgeführt werden, während die Veresterung mit Maleinsäureanhydrid, Bernsteinsäureanhydrid oder Phthalsäureanhydrid bei 0°C bis 150°C, im Falle von Maleinsäureanhydrid vorzugsweise bei 40 bis 80°C und im Falle von Bernsteinsäureanhydrid und Phthalsäureanhydrid vorzugsweise bei 90 bis 120°C erfolgt.

Als saure Katalysatoren sind organische und anorganische Säuren, beispielsweise Benzolsulfonsäure, p-Toluolsulfonsäure, Borsäure und Schwefelsäure, Lewissäuren wie Zinkchlorid oder Zinnpulver sowie Gemische davon oder saure Ionenaustauscher geeignet. Es ist zweckmäßig, die organischen und anorganischen Säuren sowie die Lewissäuren in einer Konzentration von 0,05 bis 3 Gew.-%, vorzugsweise 0,5 bis 1 Gew.-%, Zinnpulver in einer Konzentration von 2 bis 5 Gew.-%, vorzugsweise 3 bis 4 Gew.-%, und den sauren Ionenaustauscher in einer Konzentration von 3 bis 10 Gew.-%, vorzugsweise 5 bis 7 Gew.-%, jeweils bezogen auf das zu veresternde Oxalkylat, einzusetzen.

Die Veresterung der unmodifizierten oder modifizierten natürlichen Harzsäuren mit einem mehrwertigen Alkohol oder mit einem Aminooxalkylat aus 1 bis 5 Einheiten der Verbindung der Formel (II) erfolgt in einem solchen Molverhältnis, daß das jeweilige Veresterungsprodukt noch 1 bis 11, vorzugsweise 2 bis 6, freie Hydroxygruppen aufweist. Vorzugsweise wird das Molverhältnis 1:1 bis 3:1 von Harzsäure zu mehrwertigem Alkohol oder Aminooxalkylat verwendet. Die Veresterungsreaktion kann nach üblichen Verfahren bei etwa 100 bis 300°C, vorzugsweise bei 160 bis 270°C, gegebenenfalls unter Zusatz eines Schleppmittels, beispielsweise eines aromatischen Kohlenwasserstoffs oder Chlorkohlenwasserstoffs, gegebenenfalls auch unter Zusatz eines Katalysators wie Benzolsulfonsäure, p-Toluolsulfonsäure, Borsäure, Schwefelsäure, Salzsäure und Zinnpulver in den vorstehend angegebenen Konzentrationen durchgeführt werden.

Die Oxalkylierung der unmodifizierten oder modifizierten natürlichen Harzsäuren, Harzalkohole, Harzamine und Harzsäureester, von denen sich der Rest A in der allgemeinen Formel (I) ableitet, kann nach üblichen Methoden erfolgen. Vorzugsweise wird der jeweilige Harzkörper bei einer Temperatur von 100 bis 200°C, vorzugsweise bei 120 bis 160°C, mit Ethylenoxid oder Propylenoxid oder beiden Epoxiden (abwechselnd oder als Gemisch) in Gegenwart eines Hydroxids oder eines Alkoxylats als Katalysator, vorzugsweise eines Alkalihydroxids, wie Kaliumhydroxid oder insbesondere Natriumhydroxid, oder eines Alkalialkoxylats, wie Natriummethylat oder Natriumethylat, umgesetzt. Die Menge der verwendeten Epoxide oder des Epoxidgemisches wird so bemessen, daß pro reaktionsfähiges Wasserstoffatom der freien Carboxygruppen, Hydroxygruppen und Aminogruppen des jeweiligen Harzkörpers 1 bis 100 Mol, vorzugsweise 2 bis 20 Mol, des oder der verwendeten Epoxide angelagert werden. Die Konzentration des Katalysators Alkalihydroxid oder Alkalialkoxylat soll bevorzugt 0,05 bis 1,0 Gew.-%, bezogen auf den Harzkörper, bei Beginn der Oxalkylierung betragen. Die Oxalkylierung kann drucklos oder in Druckgefäßen mit Propylenoxid oder bevorzugt mit Ethylenoxid oder mit Mischungen von beiden Epoxiden durchgeführt werden, wobei das Alkylenoxid gasförmig oder flüssig zugeführt werden kann. Der Arbeitsdruck beträgt in der Regel 1 bis 10 bar, vorzugsweise 2 bis 8 bar. Die Menge des angelagerten Alkylenoxids kann je nach dem beabsichtigten Einsatzzweck und dem angestrebten Grad der Hydrophilie der aus den Oxalkylierungsprodukten hergestellten Aminkondensate oder Aminsalze der Formel (I) variiert und optimiert werden.

Die Umsetzung der Formel Z zugrundeliegenden Amine mit dem im vorangehenden Reaktionsschritt hergestellten oxalkylierten Harzkörper erfolgt nach Halbveresterung der endständigen Hydroxygruppen der oxalkylierten Harzkörper mit geeigneten Dicarbonsäuren oder deren Anhydriden. Für die Veresterung sind beispielsweise geeignet: Maleinsäure, Maleinsäureanhydrid, Fumarsäure, Phthalsäure, Phthalsäureanhydrid, Isophthalsäure, Terephthalsäure, Oxalsäure, Malonsäure, Bernsteinsäure, Bernsteinsäureanhydrid, Glutarsäure, Glutarsäureanhydrid, Adipinsäure, Pimelinsäure, Korksäure, Acelainsäure, Sebacinsäure.
Die Halbveresterung der Oxalkylate mit den genannten Dicarbonsäuren erfolgt nach üblichen Verfahren bei 130 bis 220°C, vorzugsweise bei 150 bis 180°C, in Gegenwart von anorganischen oder organischen Säuren oder von Lewissäuren wie Zinkchlorid, Benzolsulfonsäure, p-Toluolsulfonsäure, Borsäure, Zinnpulver oder Schwefelsäure in den vorstehend angegebenen Konzentrationen. Das sich dabei bildende Reaktionswasser wird gegebenenfalls unter Zusatz eines Schleppmittels, beispielsweise eines Kohlenwasserstoffes oder Chlorkohlenwasserstoffes, durch Abdestillieren entfernt. Bei Verwendung von Dicarbonsäureanhydriden erfolgt die Halbveresterung schon bei 0 bis 150°C, vorzugsweise bei 40 bis 120°C, gegebenenfalls in Anwesenheit von Alkalihydroxiden, wobei die Konzentration der Alkalihydroxide 0,1 bis 1,0 Gew.-%, bezogen auf die Gesamtmischung betragen soll. Im Falle von Maleinsäureanhydrid, Bernsteinsäureanhydrid und Phthalsäureanhydrid ist es wegen der Sublimationsneigung vorteilhaft, in Druckgefäßen unter einem Überdruck von 0,2 bis 1,0 bar Stickstoff oder Luft zu arbeiten und für kräftiges Durchmischen zu sorgen, da zu Beginn der Reaktion die geschmolzenen Anhydride mit den oxalkylierten Harzverbindungen nur in geringem Maße mischbar sind.
Die mit einer Carboxylgruppe versehenen Oxalkylierungsprodukte werden dann mit den Z zugrundeliegenden Aminen entweder zu Amiden oder zu Aminsalzen umgesetzt, wobei pro freie Carboxylgruppe des jeweiligen Halbesters die etwa equimolare Menge des Amins eingesetzt wird. Im Falle der Amidierung liegt die einzuhaltende Reaktionstemperatur üblicherweise zwischen 20 bis 240°C, vorzugsweise jedoch bei 130 bis 180°C, je nach Amidierungsmethode. Bevorzugt wird zur Erhöhung der Ausbeute die Amidierung in einem inerten organischen Lösungsmittel durchgeführt, das als Schleppmittel zum Entfernen des Reaktionswassers geeignet ist. Beispielsweise kann die Amidierung in Xylol als organischem Lösungsmittel und in Gegenwart von sauren Katalysatoren bei einer Temperatur von 130 bis 220°C durchgeführt werden. Als saure Katalysatoren sind beispielsweise Säuren und Lewissäuren, wie Benzolsulfonsäure, p-Toluolsulfonsäure, Borsäure, Zinnpulver, Zinkchlorid oder Schwefelsäure geeignet.
Hingegen erfolgt die Herstellung der Aminsalze bei einer Temperatur von 20 bis 130°C, vorzugsweise bei 40 bis 90°C, insbesondere bei 70 bis 90°C.

Die Ausbeute an Endprodukt beträgt sowohl bei der Amidierung als auch bei der Herstellung des Aminsalzes mehr als 90 % der theoretischen Wertes.

Beispiele für die Z zugrundeliegenden Amine, wobei Z für die genannten Formeln (IIIa) und (IIIb) steht, sind Alkylendiamine und Polyamine der Formel H[-NH-(CR⁷R⁸)ᵤ]_{w}-NH₂, wobei R⁷, R⁸, u und w die in den Formeln (IIIa) und (IIIb) genannten Bedeutungen haben.
Derartige Verbindungen sind beispielsweise 1,2-Diaminoethan, 1,3-Diaminopropan, 1,2-Diaminopropan, 1,4-Diaminobutan, 1,5-Diaminopentan, 1,6-Diaminohexan, 1,7-Diaminoheptan, 1,8-Diaminooctan, 1,9-Diaminononan, 1,10-Diaminodecan, 1,12-Diaminododecan, Diethylentriamin, Dipropylentriamin, Triethylentetramin, Dipropylentetramin, Tetraethylenpentamin, Tetrapropylenpentamin, Pentaethylenhexamin, Pentapropylenhexamin, Hexaethylenheptamin, Hexapropylenheptamin, Heptaethylenoctamin, Heptapropylenoctamin, 1,3-Diamino-2,2-dimethyl-propan, 1,2-Diamino-2-methyl-propan, 1,3-Diamino-2-methyl-propan, 2,5-Diamino-2,5-dimethylhexan, N-(2-amino-ethyl)-1,3-propylendiamin und N,N'-bis-(3-aminopropyl)ethylendiamin.

Weiterhin sind Amine der Formel H[-NH-(CH₂)ᵤ-]_{w}-NH-CH₂CH₂OH geeignet, wobei u und w die in den Formeln (IIIa) und (IIIb) genannten Bedeutungen haben, beispielsweise Alkylolamine, wie N-(2-Hydroxyethyl)-1,2-diaminoethan, N-(2-Hydroxyethyl)-diethylentriamin, N-(2-Hydroxyethyl)-triethylentetramin und 2-Amino-ethanol.

Gegenstand der Erfindung ist auch die Verwendung der erfindungsgemäßen Verbindungen als grenzflächenaktives Mittel. Die erfindungsgemäßen Substanzen besitzen aufgrund ihrer vielseitigen oberflächenaktiven Eigenschaften ein breites Anwendungsspektrum. Sie gehören zu der Klasse der oberflächenaktiven Verbindungen nach DIN 53 900, senken die Oberflächenspannung nach der Ringabreißmethode (DIN 53 914) und sind nach den Resultaten im modifizierten Ross-Miles-Test (DIN 53 902) als nichtschäumende oder schwachschäumende, oberflächenaktive Stoffe zu bezeichnen. Bei geeignetem Hydrophilierungsgrad zeigen sie ausgezeichnetes Netzvermögen für Baumwolle nach der Tauchnetzmethode (DIN 53 901) bei gleichzeitig gutem Egalisierverhalten nach DIN 53 504. Vorteilhaft ist daher der Einsatz als Netz-, Egalisier- und Färbereihilfsmittel. Sie besitzen weiterhin ein sehr gutes Flockungsschutzvermögen (DIN 53 908) gegenüber Pigmenten und Farbstoffen und können darüber hinaus als Retentionsmittel verwendet werden.

Die erfindungsgemäßen Substanzen können sowohl als Emulgatoren als auch als Dispergiermittel für Feststoffdispersionen verwendet werden. Dies gilt vor allem für die Anwendung als Kupplungshilfsmittel oder Präparationsmittel oder beides bei der Herstellung von Azoverbindungen, vorzugsweise Azofarbmitteln, insbesondere von Azopigmenten für den Offsetdruck, als Dispergiermittel für die Feinverteilung und Stabilisierung von schwerlöslichen oder unlöslichen Farbmitteln und für die Herstellung von gut fließfähigen Pigmentdispersionen.

Die erfindungsgemäßen Substanzen eignen sich ferner als Additive und Emulgatoren für die Herstellung von Fett- und Mineralölemulsionen, beispielsweise als Korrosionsschutzzusätze, und als Zusätze für die Herstellung von Kühlschmiermitteln und Kaltwalzölen in der Metallverarbeitungsindustrie.

Die erfindungsgemäßen Verbindungen können einzeln oder als Gemische sowie in Kombinationen mit anderen nichtionogenen oder kationaktiven Tensiden oder Gemischen davon eingesetzt werden. Weiterhin können sie zusammen mit üblichen Mengen an Gerüstsubstanzen oder anderen üblichen Zusätzen oder Hilfsstoffen in Emulgier- und Dispergiermittelformulierungen zur Anwendung kommen.

In den folgenden Beispielen beziehen sich "Teile" und Prozentangaben auf das Gewicht, Volumenteile verhalten sich zu Gewichtsteilen wie Kilogramm zu Liter. Druckangaben bedeuten "Überdruck", bezogen auf Atmosphärendruck, sofern nichts anderes angegeben ist. Die Säurezahl (SZ) wird nach DIN 53 402 bestimmt. Die Säurezahl gibt die Menge an Kaliumhydroxid in Milligramm an, die zur Neutralisation von 1 g des Reaktionsprodukts verbraucht wird.
Die Hydroxylzahl wird nach DIN 53 240 bestimmt und gibt die Menge an Kaliumhydroxid in Milligramm an, die zur Umsetzung von 1 g des Reaktionsprodukts verbraucht wird. Die Aminzahl wird nach DIN 53 176 bestimmt und ist diejenige Menge an Kaliumhydroxid in Milligramm, die dem Aminanteil von 1 g Substanz äquivalent ist.

### Herstellungsbeispiel 1

### a) Herstellung des Harzsäure-Oxethylates

302 Teile Kolophonium wurden nach Zugabe von 2,2 Teilen Natriummethylat in einem Druckgefäß unter Rühren und Zufuhr von 440 Teilen Ethylenoxid bei 150 bis 160°C unter Aufrechterhaltung eines Druckes von 4 bis 6 bar oxalkyliert. Nachdem das ganze Ethylenoxid aufgedrückt war, wurde eine Stunde bei 150 bis 160°C nachgerührt. Das so erhaltene Harzsäureaddukt ist braun, dickflüssig und enthält im Mittel 10 Ethylenoxy-Einheiten pro Molekül bei einer Hydroxylzahl nach DIN 53 240 von 75,0.

### b) Herstellung des Malonsäureesters:

300 Teile Harzsäure-Oxethylat a) wurden mit 42,0 Teilen Malonsäure auf 70 bis 80°C erwärmt und unter Stickstoffgas eine Stunde lang gerührt. Nach Zugabe von 1,5 Teilen p-Toluolsulfonsäure und 150 Teilen Xylol wurde 16 Stunden lang auf 150 bis 160°C erhitzt, wobei das Reaktionswasser durch Auskreisen am Wasserabscheider entfernt wurde. Danach wurde das Xylol abdestilliert und die Säurezahl nach DIN 53 402 bestimmt. Das Produkt hat eine Säurezahl von 72,3.

### c) Herstellung des Harzaminkondensates:

300 Teile Malonsäureester b) wurden vorgelegt und mit 40,2 Teilen N-(2-Hydroxyethyl)-1,2-diaminoethan entsprechend der Säurezahl, bei 60 bis 70°C gut vermischt. Nach Zugabe von 0,5 Teilen p-Toluolsulfonsäure und 150 Teilen Xylol wurde analog der Veresterung b) bis zu einer Säurezahl unter 20 amidiert. Man erhielt ein dunkelbraunes, wachsweiches Produkt mit einer Aminzahl zwischen 115 und 120.

### Herstellungsbeispiel 2

### a) Herstellung des Harzalkohol-Oxethylates:

292 Teile technischer Hydroabietylalkohol (Firma Hercules, USA) wurden nach Zugabe von 1 Teil Kaliumhydroxid in einem Druckgefäß unter Rühren und Zuführen von 528 Teilen Ethylenoxid bei 150 bis 160°C unter Aufrechterhaltung eines Druckes von etwa 1,5 bis 2 bar oxalkyliert. Nachdem das ganze Ethylenoxid aufgedrückt war, wurde eine Stunde bei 150 bis 160°C nachgerührt. Das erhaltene Harzalkoholaddukt enthält im Mittel 12 Ethylenoxy-Einheiten pro Molekül und eine Hydroxylzahl zwischen 65 und 70.

### b) Herstellung des Bernsteinsäurehalbesters:

300 Teile Harzalkohol-Oxethylat a) wurden auf 50 bis 60°C erwärmt und portionsweise 36,76 Teile Bernsteinsäureanhydrid eingetragen. Nach Erhöhung der Reaktionstemperatur auf 100 bis 120°C wurde unter Stickstoffüberlagerung 3 bis 4 Stunden nachgerührt. Der erhaltene Bernsteinsäurehalbester enthält im Mittel eine Carboxylgruppe im Molekül mit einer durchschnittlichen Säurezahl von 63.

### c) Herstellung eines Diethylentriaminsalzes:

300 Teile Bernsteinsäurehalbester b) wurden bei anfänglich 60 bis 70°C durch Zugabe einer Lösung aus 34,7 Teilen Diethylentriamin und 502,1 Teilen entmineralisiertem Wasser innerhalb von 1 bis 2 Stunden bei bis auf 20 bis 25°C fallender Reaktionstemperatur in das Aminsalz überführt. Die Menge des zugesetzten Wassers kann zwischen 50 bis 85 % des Endproduktes betragen. Das wäßrige Aminsalz besitzt bei einer Aminzahl von etwa 164, bezogen auf Feststoff, einen pH-Wert von 8,6.

### Herstellungsbeispiel 3

### a) Herstellung des Harzsäuremonoglycerinesters:

302 Teile Kolophonium wurden in Gegenwart von 4 Teilen Zinnpulver mit 92 Teilen Glycerin in einem geeigneten Rührgefäß unter Abdestillieren des Reaktionswassers innerhalb von 8 bis 10 Stunden bei 230 bis 250°C bei gleichzeitigem Durchleiten von Stickstoff bis zu einer Säurezahl (DIN 53 402) von 20 verestert.

### b) Herstellung des Harzsäureglycerinester-Oxethylates:

356 Teile Harzsäuremonoglycerinester nach a) wurden nach Zugabe von 2,7 Teilen Natriummethylat (30 %ig in Methanol) in einem Druckgefäß unter Rühren und Zuführen von 440 Teilen Ethylenoxid bei 150 bis 160°C unter Aufrechterhaltung eines Druckes von 2 bis 8 bar oxalkyliert. Nachdem das ganze Ethylenoxid aufgedrückt war, wurde noch 1 Stunde bei 150 bis 160°C gerührt. Das erhaltene Harzsäureglycerinester-oxethylat enthält im Mittel 10 Ethylenoxy-Einheiten pro Molekül und eine Hydroxylzahl zwischen 135 bis 140.

### c) Herstellung des Di-maleinsäurehalbesters:

300 Teile Harzsäureglycerinester-oxethylat b) wurden analog Herstellungsbeispiel 2b) mit 72,0 Teilen Maleinsäureanhydrid bei 75 bis 80°C innerhalb von 3 bis 4 Stunden bis zu einer Säurezahl zwischen 108 und 112 verestert.

### d) Herstellung des Harzaminkondensates:

300 Teile Di-maleinsäurehalbester c) wurden entsprechend der Säurezahl mit 61,4 Teilen N-(2-Hydroxyethylen)-1,2-diaminoethan analog Herstellungsbeispiel 1c) mit 1,5 Teilen p-Toluolsulfonsäure in 150 Teilen Xylol bei 155 bis 165°C in etwa 10 Stunden amidiert. Man erhielt ein braunes, wachsweiches Produkt mit einer Aminzahl zwischen 185 bis 195.

### Herstellungsbeispiel 4

### a) Herstellung des Harzsäure-Oxethylats:

302 Teile Kolophonium wurden entsprechend Herstellungsbeispiel 1a) nach Zugabe von 0,5 Teilen pulverförmigem Ätznatron mit 1320 Teilen Ethylenoxid umgesetzt. Das Produkt enthält im Mittel 30 Ethylenoxy-Einheiten pro Molekül und eine Hydroxylzahl zwischen 30 bis 35.

### b) Herstellung des Phthalsäurehalbesters:

500 Teile Harzsäure-Oxethylat a) wurden auf etwa 50°C erwärmt und portionsweise 43,0 Teile Phthalsäure entsprechend vorliegender Hydroxylzahl eingetragen und 3 bis 4 Stunden bei 100 bis 120°C unter Stickstoff gerührt. Man erhielt einen gelbbraunen, zähflüssigen Phthalsäurehalbester mit einer Säurezahl von 32.

### c) Herstellung eines Triethylentetraminsalzes:

300 Teile Phthalsäurehalbester wurden auf 50 bis 60°C erwärmt und entsprechend der Säurezahl durch Eintragen von 25 Teilen Triethylentetramin und Erhöhung der Reaktionstemperatur auf 65 bis 75°C in etwa 1 Stunde zum Aminsalz umgesetzt. Man erhielt ein gelbbraunes Aminprodukt mit einer Aminzahl zwischen 90 und 100.

### Herstellungsbeispiel 5

### a) Herstellung des Harzamin-Oxethylates:

286 Teile technisches Dehydroabietylamin (Amine D der Firma Hercules, USA) wurden in einem Druckgefäß mit 4,5 Teilen Natriummethylat (30 %ig in Methanol) versetzt und nach Entfernen des überschüssigen Methanols durch Zuführung von 440 Teilen Ethylenoxid bei 120 bis 140°C unter Aufrechterhaltung eines Druckes zwischen 1,5 und 2,5 bar umgesetzt. Nachdem die gesamte Menge Ethylenoxid aufgedrückt war, ließ man bei 130 bis 140°C noch 1 Stunde lang rühren. Man erhielt ein dickflüssiges, braunes Produkt mit durchschnittlich 10 Ethylenoxid-Einheiten pro Molekül und einer Hydroxylzahl von 155.

### b) Herstellung des Di-maleinsäurehalbesters:

300 Teile Harzamin-Oxethylat a) wurden analog Herstellungsbeispiel 2b) mit 81,2 Teilen Maleinsäureanhydrid bei 75 bis 80°C innerhalb von 3 bis 4 Stunden umgesetzt. Man erhielt einen gelben, wachsweichen Di-maleinsäurehalbester mit einer Säurezahl von etwa 122.

### c) Herstellung des Harzaminkondensates:

300 Teile Di-maleinsäurehalbester b) wurden entsprechend der Säurezahl gemäß Herstellungsbeispiel 1c) mit 136 Teilen N-(2-Hydroxyethyl)-1,2-diaminoethan nach Zugabe von 1,5 Teilen p-Toluolsulfonsäure und 150 Teilen Xylol bei 155 bis 165°C amidiert. Man erhielt ein braunes, wachsweiches Aminkondensat mit einer Aminzahl von etwa 200.

### Herstellungsbeispiel 6

### a) Herstellung des Harzsäuremonopentaerythritesters:

302 Teile Kolophonium wurden in Gegenwart von 4,5 Teilen Borsäure mit 136 Teilen Pentaerythrit entsprechend Herstellungsbeispiel 3a) bis zu einer Säurezahl von 15 verestert.

### b) Herstellung des Harzsäuremonopentaerythritester-Oxethylates:

420 Teile Harzsäuremonopentaerythritester a) wurden nach Zugabe von 3,1 Teilen Natriummethylat (30 % in Methanol) entsprechend dem Herstellungsbeispiel 3b) mit 660 Teilen Ethylenoxid umgesetzt. Das erhaltene Harzsäuremonopentaerythritester-Oxethylat enthält im Mittel 15 Ethylenoxy-Einheiten pro Molekül und eine Hydroxyzahl von 153.

### c) Herstellung des Tri-maleinsäurehalbesters:

300 Teile Harzsäuremonopentaerythritester-Oxethylat b) mit einer Hydroxylzahl von 153 wurden entsprechend Herstellungsbeispiel 2b) mit 80 Teilen Maleinsäureanhydrid bei 75 bis 80°C innerhalb von 4 Stunden verestert. Man erhielt ein gelbes, wachsartiges Veresterungsprodukt mit einer Säurezahl zwischen 120 und 125.

### d) Herstellung eines Tri-1,2-ethylendiaminsalzes:

300 Teile Tri-maleinsäurehalbester c) wurden durch portionsweise Zugabe von 39 Teilen 1,2-Ethylendiamin (berechnet auf Säurezahl) bei 60 bis 70°C innerhalb von 1 bis 1,5 Stunden umgesetzt. Man erhielt ein gelbbraunes, wachsartiges Produkt mit einer Aminzahl zwischen 195 und 210.

### Herstellungsbeispiel 7

### a) Herstellung des Harzamin-Oxethylats:

286 Teile technisches Dehydroabietylamin (Amine D der Firma Hercules, USA) wurden vorgelegt und bei 120 bis 140°C und einem Druck von 3 bis 4 bar mit 232 Teilen Propylenoxid umgesetzt. Nach 1 Stunde Nachrührzeit wurden bei gleicher Temperatur und gleichen Druckbedingungen nach Zugabe von 6 Teilen Natriummethylat (30 %ig in Methanol) und Entfernung des Methanols 264 Teile Ethylenoxid zugeführt und nochmals 1 Stunde bei 140 bis 145°C und einem Druck von 3 bis 4 bar nachgerührt. Man erhielt ein dickflüssiges gelbbraunes Harzamin-Oxalkylat, das im Mittel 4 Propylenoxid-Einheiten und 6 Ethylenoxid-Einheiten pro Molekül enthält. Die Hydroxylzahl des Oxalkylates liegt zwischen 140 und 150.

### b) Herstellung des Di-maleinsäurehalbesters:

300 Teile Harzamin-Oxalkylat a) wurden analog Herstellungsbeispiel 2b) gemäß Hydroxylzahl 143,5 mit 75,2 Teilen Maleinsäureanhydrid bei 75 bis 80°C innerhalb von 3 bis 4 Stunden verestert. Man erhielt ein gelbes Halbesterprodukt mit einer Säurezahl von 115.

### c) Herstellung eines Diethylentriaminsalzes:

300 Teile Di-maleinsäurehalbester b) wurden gemäß Herstellungsbeispiel 6d) mit 63,9 Teilen Diethylentriamin innerhalb von 1 bis 1,5 Stunden umgesetzt. Das erhaltene Aminprodukt besitzt einen pH-Wert zwischen 8,9 und 9,3 und eine Aminzahl von etwa 286.

### Herstellungsbeispiel 8

### a) Herstellung des Diaminopropan-Oxalkylats:

50 Teile 1,3-Diaminopropan wurden unter Rühren und Zuführen von 162 Teilen Propylenoxid bei 90 bis 110°C umgesetzt und nach Zugabe von 3 Teilen Natriummethylat (30 % in Methanol) und Entfernen des Methanols unter reduziertem Druck mit 982,8 Teilen Ethylenoxid bei 120 bis 140°C bei 3 bis 5 bar oxethyliert. Das erhaltene Oxalkylat enthält pro Molekül 4 Propylenoxid-Einheiten und 20 Ethylenoxid-Einheiten. Das vorliegende Oxalkylat besitzt eine Hydroxylzahl von 191,5.

### b) Herstellung des Di-Harzsäureesters:

300 Teile 1,3-Diaminopropan-Oxalkylat a) wurden mit 155 Teilen disproportioniertem Kolophonium in Gegenwart von 6 Teilen Zinnpulver und 1,5 Teilen p-Toluolsulfonsäure gemäß Herstellungsbeispiel 3a) bei 155 bis 165°C innerhalb von 10 Stunden verestert. Der erhaltene braune, dickflüssige Harzester besitzt eine Hydroxylzahl von 64,4.

### c) Herstellung des Di-maleinsäurehalbesters:

400 Teile Di-Harzsäureester b) wurden entsprechend Hydroxylzahl mit 44,9 Teilen Maleinsäureanhydrid analog Herstellungsbeispiel 3a) bei 75 bis 80°C innerhalb von 3 bis 4 Stunden bis zu einer Säurezahl von 57 verestert.

### d) Herstellung eines Diethylentriaminsalzes:

300 Teile Di-maleinsäurehalbester c) wurden gemäß der Säurezahl entsprechend Herstellungsbeispiel 2c) mit einer Lösung aus 31,7 Teilen Diethylentriamin und 487,5 Teilen entmineralisiertem Wasser innerhalb von 1 bis 1,5 Stunden umgesetzt. Die Menge des zugesetzten Wassers kann zwischen 50 bis 85 % des Endproduktes betragen. Das vorliegende Aminsalz besitzt einen pH-Wert von 8,2 und eine Aminzahl von etwa 210.

### Herstellungsbeispiel 9

### a) Herstellung des 1,2-Diaminoethan-Oxalkylates:

50 Teile 1,2-Diaminoethan wurden unter Rühren und Zuführen von 195 Teilen Propylenoxid bei 90 bis 110°C oxpropyliert und nach Zugabe von 3 Teilen Natriumethylat (30 %ig in Methanol) und Entfernung des Methanols unter reduziertem Druck mit 880 Teilen Ethylenoxid bei 120 bis 140°C und 3 bis 5 bar umgesetzt. Das erhaltene Oxalkylat enthält pro Molekül 4 Propylenoxid-Einheiten und 24 Ethylenoxid-Einheiten und besitzt eine Hydroxylzahl von etwa 163.

### b) Herstellung eines 3fachen verknüpften Aminooxalkylates:

600 Teile 1,2-Diaminoethan-Oxalkylat a) wurden entsprechend Hydroxylzahl von 163 mit 30,9 Teilen Malonsäure nach Zugabe von 3 Teilen p-Toluolsulfonsäure und 200 Teilen Xylol als Schleppmittel 10 Stunden bei 155 bis 165°C erhitzt und das Reaktionswasser durch Auskreisen entfernt. Nach Abdestillieren des Xylols hatte das Produkt eine Säurezahl von weniger als 10, eine Hydroxylzahl von etwa 106 und pro Molekül einen Gehalt von 12 Propylenoxid- und 72 Ethylenoxid-Einheiten.

### c) Herstellung eines 4fachen Harzesters:

400 Teile des 3fachen verknüpften Aminooxalkylates b) wurden entsprechend einer Hydroxylzahl von 53 mit 114 Teilen disproportioniertem Kolophonium entsprechend Herstellungsbeispiel 3a) bis zu einer Säurezahl von etwa 10 verestert.

### d) Herstellung eines 4fachen Maleinsäurehalbesters:

200 Teile Harzsäure-Aminooxalkylatester c) wurden nach der noch vorhandenen Hydroxylzahl von 26 mit 18,5 Teilen Maleinsäureanhydrid innerhalb von 3 bis 4 Stunden bei 75 bis 85°C halbverestert und anschließend entsprechend einer Säurezahl von 76,6 mit einer Lösung aus 31 Teilen Diethylentriamin und 374,3 Teilen Wasser in 1 bis 1,5 Stunden bei von 60°C bis auf 20 bis 25°C fallender Temperatur umgesetzt. Man erhielt eine gelbbraune Aminsalzlösung mit einem pH-Wert von etwa 8,5 und einer Aminzahl von etwa 200. Die Menge des zugesetzten Wassers betrug vorzugsweise zwischen 50 bis 80 % der fertigen Lösung des Produktes.

### Herstellungsbeispiel 10

### a) Herstellung des Diethylentriamin-Oxalkylates:

50 Teile Diethylentriamin wurden analog Herstellungsbeispiel 8a) mit 281,3 Teilen Propylenoxid und nach Zugabe von 2,5 Teilen Natriummethylat (30 %ig in Methanol) und Entfernung des Methanols unter reduziertem Druck mit 853,3 Teilen Ethylenoxid umgesetzt. Das hellgelbe Oxalkylat enthielt pro Molekül 10 Propylenoxid-Einheiten und 40 Ethylenoxid-Einheiten bei einer Hydroxylzahl von etwa 115.

### b) Herstellung des 2fachen Harzesters:

300 Teile Diethylentriamin-Oxalkylat a) wurden analog Herstellungsbeispiel 3a) mit 73,6 Teilen disproportioniertem Kolophonium bis zu einer Säurezahl unter 10 verestert. Die Hydroxylzahl des Produkts beträgt 55.

### c) Herstellung des Tri-maleinsäurehalbesters:

300 Teile des 2fachen Harzesters b) wurden gemäß Herstellungsbeispiel 3c) entsprechend Hydroxylzahl mit 29,2 Teilen Maleinsäureanhydrid innerhalb von 3 bis 4 Stunden bei 75 bis 80°C verestert. Der erhaltene braune, zahflüssige Halbester besitzt eine Säurezahl von etwa 170.

### d) Herstellung eines 1,2-Ethandiaminsalzes:

400 Teile Tri-maleinsäurehalbester c) wurden analog Herstellungsbeispiel 8d) entsprechend der Säurezahl mit einer Lösung aus 72,7 Teilen 1,2-Ethandiamin in 709 Teilen entmineralisiertem Wasser umgesetzt. Die Menge des zugesetzten Wassers kann zwischen 50 und 85 % der fertigen Lösung des Produkts betragen. Das wäßrige Aminsalz besitzt bei einer Aminzahl von etwa 143 einen pH-Wert von 8,8.

### Herstellungsbeispiel 11

### Herstellung eines Harzaminkondensates:

500 Teile Harzamin-Oxalkylat entsprechend Herstellungsbeispiel 8a) wurden gemäß der Hydroxylzahl von 143,5 mit 125,3 Teilen Maleinsäureanhydrid bei 75 bis 80°C innerhalb von 3,5 bis 4 Stunden bis zu einer Säurezahl von 118 verestert. Anschließend wurden auf Säurezahl berechnet bei gleicher Temperatur 136,8 Teile N-(2-Hydroxyethyl)-1,2-diaminoethan innerhalb von 30 bis 60 Minuten zugemischt und gut verrührt. Nach Zugabe von 1,5 Teilen p-Toluolsulfonsäure und 200 ml Xylol und Steigerung der Reaktionstemperatur auf 155 bis 165°C wurde innerhalb von 8 bis 12 Stunden das Reaktionswasser durch Auskreisen entfernt. Danach wurde das Xylol unter Vakuum abdestilliert. Man erhielt ein dunkelbraunes, wachsweiches Produkt mit einer Säurezahl unter 25 und einer Aminzahl von 238.

### Anwendungsbeispiel 1

60,2 Teile 1-Acetoacetylamino-2,4-dimethylbenzol und 6,1 Teile 1-Acetoacetylamino-2,5-dimethoxy-4-chlorbenzol wurden in 900 Teilen Wasser und 31 Volumenteilen 33 %iger Natronlauge gelöst und nach Zusatz von 1,5 Teilen eines Fettalkoholpolyglykolethers mit Hilfe von 22 Volumenteilen Essigsäure gefällt. Nach Zugabe von 2,5 Teilen des Produkts aus Herstellungsbeispiel 7 kuppelte man mit einer Lösung von tetrazotiertem 4,4'-Diamino-3,3'-dichlordiphenyl, wobei die Tetrazoniumsalzlösung durch Zugabe von 60 Volumenteilen wäßriger 5 normaler Natriumnitritlösung zu einem Gemisch aus 38 Teilen 4,4'-Diamino-3,3'-dichlordiphenyl, 183 Volumenteilen 5 normaler Salzsäure und 520 Teilen Wasser hergestellt wurde. Nach beendeter Kupplung wurde die Pigmentsuspension mit 2,5 Teilen Dehydroabietylamin versetzt, alkalisch gestellt, anschließend mit einer Lösung, die 1,8 Teile Dimethylcocosfettaminoxid und 36 Teile eines partiell hydrierten Kolophoniums enthielt, versetzt und 30 Minuten bei 98°C erhitzt. Danach stellte man mit Salzsäure auf pH 4 und erhitzte weitere 30 Minuten bei 98°C. Anschließend wurde filtriert, gewaschen und getrocknet. Man erhielt eine Pigmentpräparation, die beim Einarbeiten in einen Druckfarbenfirnis für den Buch- und Offsetdruck eine Druckfarbe mit sehr guten anwendungstechnischen Eigenschaften ergab. Die Druckfarbe zeichnet sich im Vergleich zu einer Druckfarbe, die ohne den Zusatz des Produkts aus dem Herstellungsbeispiel 7 hergestellt worden ist, durch ein verbessertes Fließverhalten aus.

### Anwendungsbeispiel 2

Das in Anwendungsbeispiel 1 verwendete Produkt aus Herstellungsbeispiel 7 wurde durch das Produkt aus Herstellungsbeispiel 8 ersetzt. Man erhielt eine Pigmentpräparation mit ähnlich guten Eigenschaften wie im Anwendungsbeispiel 1 beschrieben.

### Anwendungsbeispiel 3

Das in Anwendungsbeispiel 1 verwendete Produkt aus Herstellungsbeispiel 7 wurde durch das Produkt aus Herstellungsbeispiel 3 ersetzt. Man erhielt eine Pigmentpräparation mit ähnlich guten Eigenschaften wie im Anwendungsbeispiel 1 beschrieben.

### Anwendungsbeispiel 4

Das in Anwendungsbeispiel 1 verwendete Produkt aus Herstellungsbeispiel 7 wurde durch das Produkt aus Herstellungsbeispiel 11 ersetzt. Man erhielt eine Pigmentpräparation mit ähnlich guten Eigenschaften wie im Anwendungsbeispiel 1 beschrieben.

### Anwendungsbeispiel 5

32,3 Teile 1-Acetoacetylamino-2,4-dimethylbenzol und 30,1 Teile 1-Acetoacetylamino-2-methylbenzol wurden in 750 Teilen Wasser und 31 Volumenteilen 33 %iger Natronlauge gelöst und nach Zusatz von 1,5 Teilen eines Fettalkoholpolyglykolethers mit 22 Volumenteilen Essigsäure gefällt. Nach Zugabe von 2,5 Teilen des Produkts aus Herstellungsbeispiel 7 kuppelte man mit einer Lösung aus 38 Teilen tetrazotiertem 4,4'-Diamino-3,3'-dichlordiphenyl, die analog Anwendungsbeispiel 1 hergestellt ist.
Anschließend wurde die Pigmentsuspension alkalisch gestellt, mit einer Lösung aus 28 Teilen eines Balsamharzes versetzt und 1 Stunde bei 98°C erhitzt. Danach stellte man mit Salzsäure auf pH 4 und erhitzte eine weitere Stunde bei 98°C.

Danach wurde filtriert, gewaschen und getrocknet. Eine analog den Anwendungsbeispielen 1 bis 4 hergestellte Druckfarbe weist vergleichbar gute drucktechnische Eigenschaften auf, insbesondere ein verbessertes Fließverhalten im Vergleich zu einer Druckfarbe, die ohne den Zusatz der Verbindung aus Herstellungsbeispiel 7 hergestellt worden ist.

### Anwendungsbeispiel 6

Das in Anwendungsbeispiel 5 verwendete Produkt aus Herstellungsbeispiel 7 wurde durch das Produkt aus Herstellungsbeispiel 11 ersetzt. Man erhielt eine Pigmentpräparation mit ähnlich guten Eigenschaften wie im Anwendungsbeispiel 5 beschrieben.

### Anwendungsbeispiel 7

520 Teile Pigment Red 12 (Colour Index Number 12370) wurden mit 72 Teilen des Diethylentriaminsalzes nach Herstellungsbeispiel 8, 35 Teilen 1,2-Propandiol und 140 Teilen Wasser in einem Doppelmuldenkneter etwa eine Stunde zäh geknetet. Nach erfolgter Feinverteilung wurde durch Zugabe von weiteren 125 Teilen 1,2-Propandiol und 109 Teilen Wasser verdünnt. Die gut fließfähige 52 %ige Pigmentpräparation eignet sich hervorragend zum Anfärben von wäßrigen Anstrichfarben und für den Einsatz in der Papiermassefärbung.

### Anwendungsbeispiel 8

400 Teile Pigment Yellow 16 (Colour Index Number 20040) wurden mit 52 Teilen des Diethylentriaminsalzes nach Herstellungsbeispiel 8, 200 Teilen 1,2-Propandiol und 110 Teilen Wasser in einer Rührwerkskugelmühle mit 1 mm Siliquarzit-Glasperlen bis zur erforderlichen Feinverteilung gemahlen. Anschließend wurden dem Mahlgut noch 238 Teile Wasser zugesetzt und die dünnflüssige Pigmentdispersion über ein Sieb von den Mahlkörpern getrennt. Die 40 gew.-%ige Pigmentdispersion ließ sich in jedem Verhältnis mit Wasser weiterverdünnen und eignet sich besonders zum Anfärben wäßriger oder alkoholhaltiger Flexo- und/oder Tiefdruckfarben.

### Anwendungsbeispiel 9

Eine Pigmentdispersion mit ähnlich guten Eigenschaften wurde erhalten, indem die 171 Teile des Diethylentriaminsalzes nach Herstellungsbeispiel 8 durch 52 Teile des 4fachen Maleinsäurehalbesters nach Herstellungsbeispiel 9 ersetzt wurden.

### Anwendungsbeispiel 10

75 Teile Methylnaphthalin wurden mit 25 Teilen des Diethylentriaminsalzes nach Herstellungsbeispiel 7 homogen verrührt. Das Konzentrat wurde anschließend mit Wasser auf 1000 Volumenteile aufgefüllt. Man erhielt eine feindisperse Carrier-Emulsion, die bei der verwendeten üblichen Verdünnung (1:10) eine hervorragende Stabilität besitzt und über längere Zeit verwendet werden kann.

### Anwendungsbeispiel 11

70 Teile eines aromatenhaltigen Mineralöls wurden mit 20 Teilen des Diethylentriaminsalzes nach Herstellungsbeispiel 8 und 10 Teilen des Harzaminkondensates nach Herstellungsbeispiel 11 homogen verrührt und anschließend mit Wasser auf 900 Volumenteile verdünnt. Man erhielt eine feindisperse bis transparente Mineralöl-Emulsion, die nach einer Verdünnung von 1:9 neben einer hervorragenden Stabilität über längere Zeit zusätzlich Korrosionsschutzeigenschaften nach DIN 51360 besitzt.

## Patentansprüche (Patentansprüche für folgende(n) Vertragsstaat(en): BE, CH, DE, DK, FR, GB, IT, LI, NL)

1. Verbindung der Formel (I)
A[(B)ₘ-Y-Z]_{q} (I)
in der
A für den Rest einer modifizierten oder unmodifizierten natürlichen Harzsäure oder eines daraus abgeleiteten Harzamins oder Harzalkohols oder für den Rest eines Veresterungsprodukts von 1 bis 6 Einheiten der genannten Harzsäuren mit einem mehrwertigen Alkohol oder eines Veresterungsprodukts von 1 bis 12 Einheiten der genannten Harzsäuren mit einem Aminooxalkylat aus 1 bis 5 Einheiten der Verbindung nach der Formel (II) 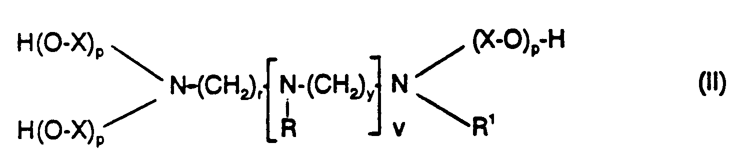
wobei das Veresterungsprodukt noch mindestens eine freie Hydroxygruppe enthält,
worin
X für eine Gruppe der Formeln -CH₂CH₂-, -CH₂CH(CH₃)- oder -CH(CH₃)CH₂- oder für eine Kombination daraus;
R für ein Wasserstoffatom oder die Gruppe -(X-O)ₚ-H
R¹ für die Gruppe -(X-O)ₚ-H oder die Gruppe -(X-O)ₚ-R², worin R² für eine divalente Gruppe -OC-E-CO steht, welche über die gezeigten freien Valenzen zwei Einheiten der Verbindung der Formel (II) esterartig verknüpft, worin E für einen divalenten aromatischen Rest mit 6 bis 12 C-Atomen oder für eine geradkettige, verzweigte oder cycloaliphatische Alkylengruppe mit 1 bis 16 C-Atomen, v für eine ganze Zahl von Null bis 4, und r und y gleich oder verschieden sind und jeweils für eine ganze Zahl von 1 bis 5 stehen;
p eine ganze Zahl von 1 bis 100, bedeutet, B eine direkte Bindung, wenn A für ein Veresterungsprodukt aus mindestens einer der obengenannten Harzverbindungen mit mindestens einem Rest einer Verbindung der Formel (II) steht,
oder eine Gruppe der Formel -(X-O)- bedeutet, worin X die obengenannte Bedeutung hat,
Y für eine Gruppe der Formeln -OC-F-CO- und -OC-F-COO⁻ steht, worin F für einen divalenten aromatischen Rest mit 6 bis 12 C-Atomen oder für eine geradkettige, verzweigte oder cycloaliphatische Alkylengruppe mit jeweils 1 bis 16 C-Atomen steht und
Z für eine Gruppe der Formel (IIIa) 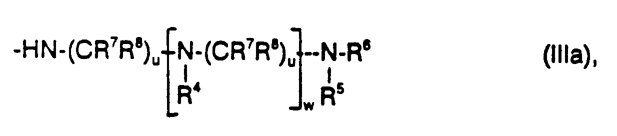 wenn Y die Bedeutung -OC-F-CO- hat
oder für ein Kation der Formel (IIIb) steht 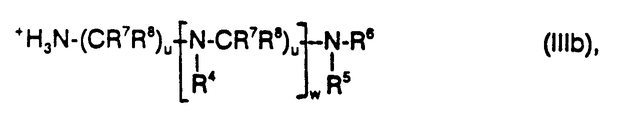 wenn Y die Bedeutung -OC-F-COO⁻ hat,
worin R⁴, R⁵ und R⁶ unabhängig voneinander für ein Wasserstoffatom oder ein Hydroxyalkylen mit 1 bis 6 C-Atomen, R⁷ und R⁸ unabhängig voneinander für Wasserstoff oder Methyl, u gleich oder verschieden ist und für eine ganze Zahl von 1 bis 14, w für eine ganze Zahl von Null bis 25, stehen und
m eine Zahl von 1 bis 100, und
q eine ganze Zahl von 1 bis 11 bedeutet.

2. Verbindung nach Anspruch 1, dadurch gekennzeichnet, daß in der Gruppe Z der Formeln (IIIa) und (IIIb)
R⁴ und R⁵ jeweils ein Wasserstoffatom,
R⁶ ein Wasserstoffatom oder ein Hydroxyalkylen mit 2 bis 3 C-Atomen bedeuten.

3. Verbindung nach Anspruch 1 oder 2, dadurch gekennzeichnet, daß in der Gruppe Z der Formeln (IIIa) und (IIIb)
R⁴, R⁵ und R⁶ jeweils Wasserstoff,
u gleich oder verschieden ist und die Zahl 2 oder 3 und
w eine ganze Zahl von Null bis 5 bedeuten.

4. Verbindung nach mindestens einem der Ansprüche 1 bis 3, dadurch gekennzeichnet, daß
Y für eine Gruppe der Formeln -OC-F-CO- und -OC-F-COO⁻ steht, worin F für einen divalenten aromatischen Rest mit 6 bis 8 C-Atomen oder für eine geradkettige oder verzweigte Alkylengruppe mit 1 bis 8 C-Atomen, vorzugsweise 1 bis 4 C-Atomen, steht.

5. Verbindung nach mindestens einem der Ansprüche 1 bis 4, dadurch gekennzeichnet, daß
B eine Gruppe der Formel -(X-O)- bedeutet, worin X für eine Gruppe der Formeln -CH₂CH₂-, -CH₂CH(CH₃)- und -CH(CH₃)-CH₂- oder für eine Kombination davon steht.

6. Verbindung nach mindestens einem der Ansprüche 1 bis 4, dadurch gekennzeichnet, daß
B eine direkte Bindung bedeutet, wenn A für ein Veresterungsprodukt, hergestellt aus mindestens einer der im Anspruch 1 genannten Harzverbindungen mit einem Aminooxalkylat aus 1 bis 5, vorzugsweise 1 bis 3, Einheiten der Verbindung gemäß Formel (II), steht.

7. Verbindung nach mindestens einem der Ansprüche 1 bis 5, dadurch gekennzeichnet, daß
A den Rest einer natürlichen Harzsäure, einer hydrierten oder disproportionierten Harzsäure, eines daraus abgeleiteten Harzamins oder Harzalkohols oder den Rest eines Veresterungsprodukts, das durch Veresterung von 1 bis 6, vorzugsweise 1 bis 2, Einheiten der vorstehend genannten Harzsäuren mit einem 2- bis 6-wertigen Alkohol unter Beibehaltung von mindestens einer freien Hydroxygruppe erhalten wird, bedeutet.

8. Verbindung nach mindestens einem der Ansprüche 1 bis 4 und 6, dadurch gekennzeichnet, daß
A ein Veresterungsprodukt, das durch Veresterung von 1 bis 12, vorzugsweise 1 bis 6, Einheiten einer natürlichen, einer hydrierten oder einer disproportionierten Harzsäure mit einem 1 bis 5, vorzugsweise 1 bis 3 Einheiten der Formel (II) enthaltenden Aminooxalkylat unter Beibehaltung von mindestens einer freien Hydroxygruppen erhalten wird, bedeutet.

9. Verbindung nach mindestens einem der Ansprüche 1 bis 8, dadurch gekennzeichnet, daß in Formel (I)
m für eine ganze Zahl von 5 bis 30 steht.

10. Verbindung nach mindestens einem der Ansprüche 1 bis 9, dadurch gekennzeichnet, daß die dem Rest A zugrundeliegende Harzverbindung eine handelsübliche Kolophoniumart ist.

11. Verfahren zur Herstellung der Verbindung nach Anspruch 1, dadurch gekennzeichnet, daß man
a) natürliche Harzsäuren, davon abgeleitete Harzalkohole oder Harzamine disproportionierte oder hydrierte Harzsäuren oder Veresterungsprodukte der genannten Harzsäuren mit mehrwertigen Alkoholen, wobei die Veresterungsprodukte noch mindestens eine freie Hydroxygruppe enthalten, mit Ethylenoxid oder Propylenoxid oder beiden Epoxiden hintereinander oder einem Gemisch beider Epoxide oxalkyliert, wobei pro reaktionsfähigem Wasserstoffatom der verwendeten Harzverbindung 1 bis 100 Mol an Epoxid eingesetzt werden, oder Oxalkylierungsprodukte, die man durch Veresterung der genannten Harzsäuren mit Aminooxalkylaten der Formel (II) erhält,
b) mit Dicarbonsäuren oder Dicarbonsäureanhydriden an den endständigen Hydroxygruppen halbverestert und
c) anschließend die freien Carboxylgruppen der entstandenen Carbonsäureverbindung A[(B)ₘ-CO-F-COOH]_{q} mit mindestens einem der Formel Z zugrundeliegenden Diamin oder Polyamin in die jeweilige Amidform oder Salzform überführt.

12. Verfahren nach Anspruch 11, dadurch gekennzeichnet, daß man als Harzverbindung eine natürliche Harzsäure, einen davon abgeleiteten Harzalkohol oder ein Harzamin oder ein Veresterungsprodukt der genannten Harzsäuren mit einem 2- bis 6-wertigen Alkohol einsetzt, wobei das Veresterungsprodukt noch mindestens eine freie Hydroxygruppe enthält.

13. Verfahren nach Anspruch 11, dadurch gekennzeichnet, daß man als Harzverbindung ein Veresterungsprodukt der in Anspruch 11 genannten Harzsäuren mit einem Aminooxalkylat der in Anspruch 1 genannten Formel (II) einsetzt, wobei das Veresterungsprodukt noch mindestens eine freie Hydroxygruppe enthält.

14. Verfahren nach mindestens einem der Ansprüche 11 bis 13, dadurch gekennzeichnet, daß die entstandene Carbonsäureverbindung A[(B)ₘ-CO-F-COOH]_{q} mit mindestens einem dem Rest Z zugrundeliegenden Amin amidiert wird.

15. Verfahren nach mindestens einem der Ansprüche 11 bis 13, dadurch gekennzeichnet, daß die entstandene Carbonsäureverbindung A[(B)ₘ-CO-F-COOH]_{q} mit mindestens einem dem Rest Z zugrundeliegenden Amin zum Aminsalz umgesetzt wird.

16. Verwendung einer nach mindestens einem der Ansprüche 1 bis 10 definierten Verbindung als grenzflächenaktives Mittel.

17. Verwendung nach Anspruch 16, dadurch gekennzeichnet, daß die Verbindung als Kupplungshilfsmittel, Emulgator, Dispergiermittel, Präparationsmittel für Feststoffdispersionen, insbesondere Farbmitteldispersionen, Korrosionsschutz- und Metallbearbeitungsmittel sowie als Netz- und Färbereihilfsmittel eingesetzt wird.

18. Verwendung nach Anspruch 16 als Kupplungshilfsmittel und Präparationsmittel bei der Herstellung von Azofarbmitteln für den Offsetdruck.

## Patentansprüche (Patentansprüche für folgende(n) Vertragsstaat(en): ES)

1. Verfahren zur Herstellung einer Verbindung der Formel (I)
A[(B)ₘ-Y-Z]_{q} (I)
in der
A für den Rest einer modifizierten oder unmodifizierten natürlichen Harzsäure oder eines daraus abgeleiteten Harzamins oder Harzalkohols oder für den Rest eines Veresterungsprodukts von 1 bis 6 Einheiten der genannten Harzsäuren mit einem mehrwertigen Alkohol oder eines Veresterungsprodukts von 1 bis 12 Einheiten der genannten Harzsäuren mit einem Aminooxalkylat aus 1 bis 5 Einheiten der Verbindung nach der Formel (II) 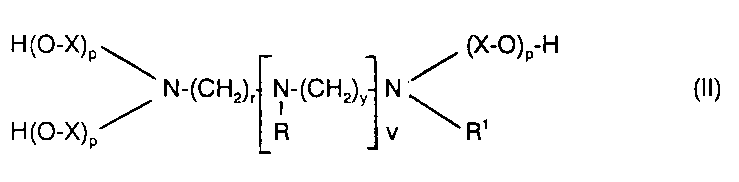
wobei das Veresterungsprodukt noch mindestens eine freie Hydroxygruppe enthält,
worin
X für eine Gruppe der Formeln -CH₂CH₂-, -CH₂CH(CH₃)- oder -CH(CH₃)CH₂- oder für eine Kombination daraus;
R für ein Wasserstoffatom oder die Gruppe -(X-O)ₚ-H
R¹ für die Gruppe -(X-O)ₚ-H oder die Gruppe -(X-O)ₚ-R², worin R² für eine divalente Gruppe -OC-E-CO- steht, welche über die gezeigten freien Valenzen zwei Einheiten der Verbindung der Formel (II) esterartig verknüpft, worin E für einen divalenten aromatischen Rest mit 6 bis 12 C-Atomen oder für eine geradkettige, verzweigte oder cycloaliphatische Alkylengruppe mit 1 bis 16 C-Atomen, v für eine ganze Zahl von Null bis 4, und r und y gleich oder verschieden sind und jeweils für eine ganze Zahl von 1 bis 5 stehen;
p eine ganze Zahl von 1 bis 100, bedeutet,
B eine direkte Bindung, wenn A für ein Veresterungsprodukt aus mindestens einer der obengenannten Harzverbindungen mit mindestens einem Rest einer Verbindung der Formel (II) steht,
oder eine Gruppe der Formel -(X-O)- bedeutet, worin X die obengenannte Bedeutung hat,
Y für eine Gruppe der Formeln -OC-F-CO- und -OC-F-COO⁻ steht, worin F für einen divalenten aromatischen Rest mit 6 bis 12 C-Atomen oder für eine geradkettige, verzweigte oder cycloaliphatische Alkylengruppe mit jeweils 1 bis 16 C-Atomen steht und
Z für eine Gruppe der Formel (IIIa) 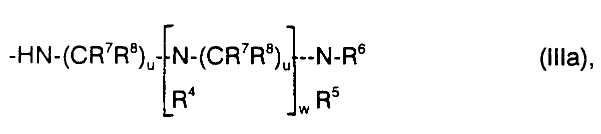 wenn Y die Bedeutung -OC-F-CO- hat
oder für ein Kation der Formel (IIIb) steht 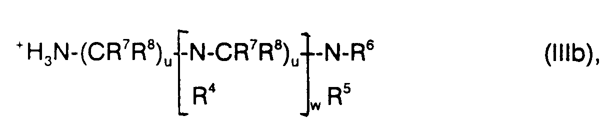 wenn Y die Bedeutung -OC-F-COO⁻ hat,
worin R⁴, R⁵ und R⁶ unabhängig voneinander für ein Wasserstoffatom oder ein Hydroxyalkylen mit 1 bis 6 C-Atomen, R⁷ und R⁸ unabhängig voneinander für Wasserstoff oder Methyl, u gleich oder verschieden ist und für eine ganze Zahl von 1 bis 14, w für eine ganze Zahl von Null bis 25, stehen und
m eine Zahl von 1 bis 100, und
q eine ganze Zahl von 1 bis 11 bedeutet,
dadurch gekennzeichnet, daß man
a) natürliche Harzsäuren, davon abgeleitete Harzalkohole oder Harzamine disproportionierte oder hydrierte Harzsäuren oder Veresterungsprodukte der genannten Harzsäuren mit mehrwertigen Alkoholen, wobei die Veresterungsprodukte noch mindestens eine freie Hydroxygruppe enthalten, mit Ethylenoxid oder Propylenoxid oder beiden Epoxiden hintereinander oder einem Gemisch beider Epoxide oxalkyliert, wobei pro reaktionsfähigem Wasserstoffatom der verwendeten Harzverbindung 1 bis 100 Mol an Epoxid eingesetzt werden, oder Oxalkylierungsprodukte, die man durch Veresterung der genannten Harzsäuren mit Aminooxalkylaten der Formel (II) erhält,
b) mit Dicarbonsäuren oder Dicarbonsäureanhydriden an den endständigen Hydroxygruppen halbverestert und
c) anschließend die freien Carboxylgruppen der entstandenen Carbonsäureverbindung A[(B)ₘ-CO-F-COOH]_{q} mit mindestens einem der Formel Z zugrundeliegenden Diamin oder Polyamin in die jeweilige Amidform oder Salzform überführt.

2. Verfahren nach Anspruch 1, dadurch gekennzeichnet, daß in der Gruppe Z der Formeln (IIIa) und (IIIb)
R⁴ und R⁵ jeweils ein Wasserstoffatom,
R⁶ ein Wasserstoffatom oder ein Hydroxyalkylen mit 2 bis 3 C-Atomen bedeuten.

3. Verfahren nach Anspruch 1 oder 2, dadurch gekennzeichnet, daß in der Gruppe Z der Formeln (IIIa) und (IIIb)
R⁴, R⁵ und R⁶ jeweils Wasserstoff,
u gleich oder verschieden ist und die Zahl 2 oder 3 und
w eine ganze Zahl von Null bis 5 bedeuten.

4. Verfahren nach mindestens einem der Ansprüche 1 bis 3, dadurch gekennzeichnet, daß
Y für eine Gruppe der Formeln -OC-F-CO- und -OC-F-COO⁻ steht, worin F für einen divalenten aromatischen Rest mit 6 bis 8 C-Atomen oder für eine geradkettige oder verzweigte Alkylengruppe mit 1 bis 8 C-Atomen, vorzugsweise 1 bis 4 C-Atomen, steht.

5. Verfahren nach mindestens einem der Ansprüche 1 bis 4, dadurch gekennzeichnet, daß
B eine Gruppe der Formel -(X-O)- bedeutet, worin X für eine Gruppe der Formeln -CH₂CH₂-, -CH₂CH(CH₃)- und -CH(CH₃)-CH₂- oder für eine Kombination davon steht.

6. Verfahren nach mindestens einem der Ansprüche 1 bis 4, dadurch gekennzeichnet, daß
B eine direkte Bindung bedeutet, wenn A für ein Veresterungsprodukt, hergestellt aus mindestens einer der im Anspruch 1 genannten Harzverbindungen mit einem Aminooxalkylat aus 1 bis 5, vorzugsweise 1 bis 3, Einheiten der Verbindung gemäß Formel (II), steht.

7. Verfahren nach mindestens einem der Ansprüche 1 bis 5, dadurch gekennzeichnet, daß
A den Rest einer natürlichen Harzsäure, einer hydrierten oder disproportionierten Harzsäure, eines daraus abgeleiteten Harzamins oder Harzalkohols oder den Rest eines Veresterungsprodukts, das durch Veresterung von 1 bis 6, vorzugsweise 1 bis 2, Einheiten der vorstehend genannten Harzsäuren mit einem 2- bis 6-wertigen Alkohol unter Beibehaltung von mindestens einer freien Hydroxygruppe erhalten wird, bedeutet.

8. Verfahren nach mindestens einem der Ansprüche 1 bis 4 und 6, dadurch gekennzeichnet, daß
A ein Veresterungsprodukt, das durch Veresterung von 1 bis 12, vorzugsweise 1 bis 6, Einheiten einer natürlichen, einer hydrierten oder einer disproportionierten Harzsäure mit einem 1 bis 5, vorzugsweise 1 bis 3 Einheiten der Formel (II) enthaltenden Aminooxalkylat unter Beibehaltung von mindestens einer freien Hydroxygruppen erhalten wird, bedeutet.

9. Verfahren nach mindestens einem der Ansprüche 1 bis 8, dadurch gekennzeichnet, daß in Formel (I)
m für eine ganze Zahl von 5 bis 30 steht.

10. Verfahren nach mindestens einem der Ansprüche 1 bis 9, dadurch gekennzeichnet, daß die dem Rest A zugrundeliegende Harzverbindung eine handelsübliche Kolophoniumart ist.

11. Verfahren nach Anspruch 1, dadurch gekennzeichnet, daß man als Harzverbindung eine natürliche Harzsäure, einen davon abgeleiteten Harzalkohol oder ein Harzamin oder ein Veresterungsprodukt der genannten Harzsäuren mit einem 2- bis 6-wertigen Alkohol einsetzt, wobei das Veresterungsprodukt noch mindestens eine freie Hydroxygruppe enthält.

12. Verfahren nach Anspruch 1, dadurch gekennzeichnet, daß man als Harzverbindung ein Veresterungsprodukt der in Anspruch 1 genannten Harzsäuren mit einem Aminooxalkylat der in Anspruch 1 genannten Formel (II) einsetzt, wobei das Veresterungsprodukt noch mindestens eine freie Hydroxygruppe enthält.

13. Verfahren nach mindestens einem der Ansprüche 1 bis 12, dadurch gekennzeichnet, daß die entstandene Carbonsäureverbindung A[(B)ₘ-CO-F-COOH]_{q} mit mindestens einem dem Rest Z zugrundeliegenden Amin amidiert wird.

14. Verfahren nach mindestens einem der Ansprüche 1 bis 12, dadurch gekennzeichnet, daß die entstandene Carbonsäureverbindung A[(B)ₘ-CO-F-COOH]_{q} mit mindestens einem dem Rest Z zugrundeliegenden Amin zum Aminsalz umgesetzt wird.

15. Verwendung einer nach mindestens einem der Ansprüche 1 bis 10 hergestellten Verbindung als grenzflächenaktives Mittel.

16. Verwendung nach Anspruch 15, dadurch gekennzeichnet, daß die Verbindung als Kupplungshilfsmittel, Emulgator, Dispergiermittel, Präparationsmittel für Feststoffdispersionen, insbesondere Farbmitteldispersionen, Korrosionsschutz- und Metallbearbeitungsmittel sowie als Netz- und Färbereihilfsmittel eingesetzt wird.

17. Verwendung nach Anspruch 15 als Kupplungshilfsmittel und Präparationsmittel bei der Herstellung von Azofarbmitteln für den Offsetdruck.

## Claims (Claims for the following Contracting State(s): BE, CH, DE, DK, FR, GB, IT, LI, NL)

1. A compound of the formula (I)
A[(B)ₘ-Y-Z]_{q} (I)
in which
A is the radical of a modified or nonmodified naturally occurring resin acid or a resin amine or resin alcohol derived therefrom, or is the radical of an esterification product of 1 to 6 units of the resin acids mentioned with a polyhydric alcohol or of an esterification product of 1 to 12 units of the resin acids mentioned with an aminooxyalkylate of 1 to 5 units of the compound according to the formula (II) 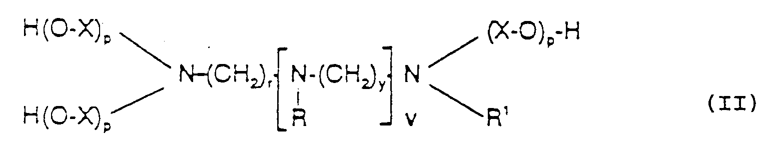
the esterification product still containing at least one free hydroxyl group,
in which
X is a group of the formula -CH₂CH₂-, -CH₂CH(CH₃)- or -CH(CH₃)CH₂- or is a combination thereof;
R is a hydrogen atom or the group -(X-O)ₚ-H,
R¹ is the group -(X-O)ₚ-H or the group -(X-O)ₚ-R², in which R² is a divalent group -OC-E-CO- which links two units of the compound of the formula (II) in ester form via the free valencies shown and in which E is a divalent aromatic radical having 6 to 12 carbon atoms or a straight-chain, branched or cycloaliphatic alkylene group having 1 to 16 carbon atoms, v is an integer from zero to 4 and r and y are identical or different and are each an integer from 1 to 5;
p is an integer from 1 to 100,
B is a direct bond, if A is an esterification product of at least one of the abovementioned resin compounds having at least one radical of a compound of the formula (II),
or is a group of the formula -(X-O)-, in which X has the abovementioned meaning,
Y is a group of the formula -OC-F-CO- or -OC-F-COO⁻, in which F is a divalent aromatic radical having 6 to 12 carbon atoms or is a straight-chain, branched or cycloaliphatic alkylene group having in each case 1 to 16 carbon atoms and
Z is a group of the formula (IIIa) 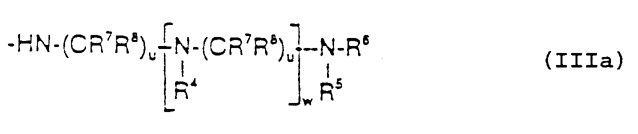 if Y is -OC-F-CO-,
or is a cation of the formula (IIIb) 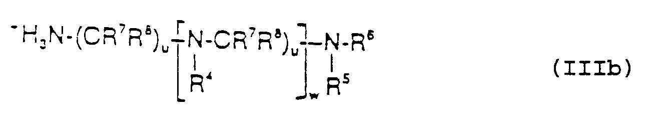 if Y is -OC-F-COO⁻,
in which R⁴, R⁵ and R⁶ independently of one another are a hydrogen atom or a hydroxyalkylene having 1 to 6 carbon atoms, R⁷ and R⁸ independently of one another are hydrogen or methyl, u is identical or different and is an integer from 1 to 14, and w is an integer from zero to 25, and
m is a number from 1 to 100 and
q is an integer from 1 to 11.

2. A compound as claimed in claim 1, wherein, in the group Z of the formula (IIIa) or (IIIb),
R⁴ and R⁵ are each a hydrogen atom and
R⁶ is a hydrogen atom or a hydroxyalkylene having 2 or 3 carbon atoms.

3. A compound as claimed in claim 1 or 2, wherein, in the group Z of the formula (IIIa) or (IIIb),
R⁴, R⁵ and R⁶ are each hydrogen,
u is identical or different and is the number 2 or 3 and
w is an integer from zero to 5.

4. A compound as claimed in at least one of claims 1 to 3, wherein
Y is a group of the formula -OC-F-CO- or -OC-F-COO⁻, in which F is a divalent aromatic radical having 6 to 8 carbon atoms or is a straight-chain or branched alkylene group having 1 to 8 carbon atoms, preferably 1 to 4 carbon atoms.

5. A compound as claimed in at least one of claims 1 to 4, wherein
B is a group of the formula -(X-O)-, in which X is a group of the formula -CH₂CH₂-, -CH₂CH(CH₃)- or -CH(CH₃)-CH₂- or is a combination thereof.

6. A compound as claimed in at least one of claims 1 to 4, wherein
B is a direct bond if A is an esterification product prepared from at least one of the resin compounds mentioned in claim 1 with an aminooxyalkylate of 1 to 5, preferably 1 to 3, units of the compound according to formula (II).

7. A compound as claimed in at least one of claims 1 to 5, wherein
A is the radical of a naturally occurring resin acid, a hydrogenated or disproportionated resin acid or a resin amine or resin alcohol derived therefrom, or the radical of an esterification product which is obtained by esterification of 1 to 6, preferably 1 to 2, units of the abovementioned resin acids with a 2- to 6-hydric alcohol, at least one free hydroxyl group being retained.

8. A compound as claimed in at least one of claims 1 to 4 and 6, wherein
A is an esterification product which is obtained by esterification of 1 to 12, preferably 1 to 6, units of a naturally occurring, a hydrogenated or a disproportionated resin acid with an aminooxyalkylate comprising 1 to 5, preferably 1 to 3, units of the formula (II), at least one free hydroxyl group being retained.

9. A compound as claimed in at least one of claims 1 to 8, wherein, in formula (I),
m is an integer from 5 to 30.

10. A compound as claimed in at least one of claims 1 to 9, wherein the resin compound on which the radical A is based is a commercially available colophony type.

11. A process for the preparation of a compound as claimed in claim 1, which comprises
a) oxyalkylating naturally occurring resin acids, resin alcohols or resin amines derived therefrom, disproportionated or hydrogenated resin acids or esterification products of the resin acids mentioned with polyhydric alcohols, the esterification products still containing at least one free hydroxyl group, with ethylene oxide or propylene oxide or both epoxides in succession or a mixture of both epoxides, 1 to 100 mol of epoxide being employed per reactive hydrogen atom in the resin compound used, or oxyalkylation products obtained by esterification of the resin acids mentioned with aminooxyalkylates of the formula (II),
b) half-esterifying the products on the terminal hydroxyl groups with dicarboxylic acids or dicarboxylic acid anhydrides and
c) subsequently converting the free carboxyl groups of the carboxylic acid compound A[(B)ₘ-CO-F-COOH]_{q} formed with at least one diamine or polyamine on which the formula Z is based into the particular amide form or salt form.

12. The process as claimed in claim 11, wherein a naturally occurring resin acid, a resin alcohol derived therefrom or a resin amine or an esterification product of the resin acids mentioned with a 2- to 6-hydric alcohol is employed as the resin compound, the esterification product still containing at least one free hydroxyl group.

13. The process as claimed in claim 11, wherein an esterification product of a resin acid mentioned in claim 11 with an aminooxyalkylate of the formula (II) mentioned in claim 1 is employed as the resin compound, the esterification product still containing at least one free hydroxyl group.

14. The process as claimed in at least one of claims 11 to 13, wherein the carboxylic acid compound A[(B)ₘ-CO-F-COOH]_{q} formed is amidated with at least one amine on which the radical Z is based.

15. The process as claimed in at least one of claims 11 to 13, wherein the carboxylic acid compound A[(B)ₘ-CO-F-COOH]_{q} formed is reacted with at least one amine on which the radical Z is based to give the amine salt.

16. The use of a compound defined in accordance with at least one of claims 1 to 10 as a surface-active agent.

17. The use as claimed in claim 16, wherein the compound is employed as a coupling auxiliary, emulsifier, dispersing agent, preparation agent for solids dispersions, in particular coloring agent dispersions, corrosion protection and metalworking agent or as a wetting agent or dyeing auxiliary.

18. The use as claimed in claim 16, as a coupling auxiliary and preparation agent in the production of azo coloring agents for offset printing.

## Claims (Claims for the following Contracting State(s): ES)

1. A process for the preparation of a compound of the formula (I)
A[(B)ₘ-Y-Z]_{q} (I)
in which
A is the radical of a modified or nonmodified naturally occurring resin acid or a resin amine or resin alcohol derived therefrom, or is the radical of an esterification product of 1 to 6 units of the resin acids mentioned with a polyhydric alcohol or of an esterification product of 1 to 12 units of the resin acids mentioned with an aminooxyalkylate of 1 to 5 units of the compound according to the formula (II) 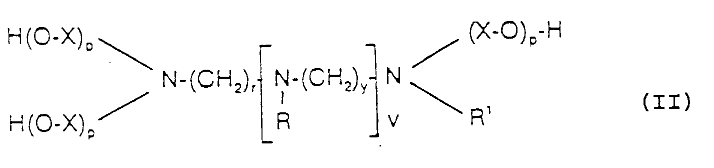
the esterification product still containing at least one free hydroxyl group,
in which
X is a group of the formula -CH₂CH₂-, -CH₂CH(CH₃)- or -CH(CH₃)CH₂- or is a combination thereof;
R is a hydrogen atom or the group -(X-O)ₚ-H,
R¹ is the group -(X-O)ₚ-H or the group -(X-O)ₚ-R², in which R² is a divalent group -OC-E-CO- which links two units of the compound of the formula (II) in ester form via the free valencies shown and in which E is a divalent aromatic radical having 6 to 12 carbon atoms or a straight-chain, branched or cycloaliphatic alkylene group having 1 to 16 carbon atoms, v is an integer from zero to 4 and r and y are identical or different and are each an integer from 1 to 5;
p is an integer from 1 to 100,
B is a direct bond, if A is an esterification product of at least one of the abovementioned resin compounds having at least one radical of a compound of the formula (II),
or is a group of the formula -(X-O)-, in which X has the abovementioned meaning,
Y is a group of the formula -OC-F-CO- or -OC-F-COO⁻, in which F is a divalent aromatic radical having 6 to 12 carbon atoms or is a straight-chain, branched or cycloaliphatic alkylene group having in each case 1 to 16 carbon atoms and
Z is a group of the formula (IIIa) 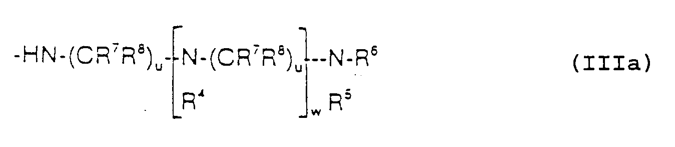 if Y is -OC-F-CO-,
or is a cation of the formula (IIIb) 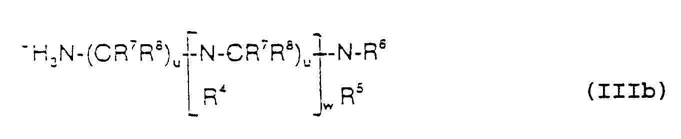 if Y is -OC-F-COO⁻,
in which R⁴, R⁵ and R⁶ independently of one another are a hydrogen atom or a hydroxyalkylene having 1 to 6 carbon atoms, R⁷ and R⁸ independently of one another are hydrogen or methyl, u is identical or different and is an integer from 1 to 14, and w is an integer from zero to 25, and
m is a number from 1 to 100 and
q is an integer from 1 to 11,
which comprises
a) oxyalkylating naturally occurring resin acids, resin alcohols or resin amines derived therefrom, disproportionated or hydrogenated resin acids or esterification products of the resin acids mentioned with polyhydric alcohols, the esterification products still containing at least one free hydroxyl group, with ethylene oxide or propylene oxide or both epoxides in succession or a mixture of both epoxides, 1 to 100 mol of epoxide being employed per reactive hydrogen atom in the resin compound used, or oxyalkylation products obtained by esterification of the resin acids mentioned with aminooxyalkylates of the formula (II),
b) half-esterifying the products on the terminal hydroxyl groups with dicarboxylic acids or dicarboxylic acid anhydrides and
c) subsequently converting the free carboxyl groups of the carboxylic acid compound A[(B)ₘ-CO-F-COOH]_{q} formed with at least one diamine or polyamine on which the formula Z is based into the particular amide form or salt form.

2. The process as claimed in claim 1, wherein, in the group Z of the formula (IIIa) or (IIIb),
R⁴ and R⁵ are each a hydrogen atom and
R⁶ is a hydrogen atom or a hydroxyalkylene having 2 or 3 carbon atoms.

3. The process as claimed in claim 1 or 2, wherein, in the group Z of the formula (IIIa) or (IIIb),
R⁴, R⁵ and R⁶ are each hydrogen,
u is identical or different and is the number 2 or 3 and
w is an integer from zero to 5.

4. The process as claimed in at least one of claims 1 to 3, wherein
Y is a group of the formula -OC-F-CO- or -OC-F-COO⁻, in which F is a divalent aromatic radical having 6 to 8 carbon atoms or is a straight-chain or branched alkylene group having 1 to 8 carbon atoms, preferably 1 to 4 carbon atoms.

5. The process as claimed in at least one of claims 1 to 4, wherein
B is a group of the formula -(X-O)-, in which X is a group of the formula -CH₂CH₂-, -CH₂CH(CH₃)- or -CH(CH₃)-CH₂- or is a combination thereof.

6. The process as claimed in at least one of claims 1 to 4, wherein
B is a direct bond if A is an esterification product prepared from at least one of the resin compounds mentioned in claim 1 with an aminooxyalkylate of 1 to 5, preferably 1 to 3, units of the compound according to formula (II).

7. The process as claimed in at least one of claims 1 to 5, wherein
A is the radical of a naturally occurring resin acid, a hydrogenated or disproportionated resin acid or a resin amine or resin alcohol derived therefrom, or the radical of an esterification product which is obtained by esterification of 1 to 6, preferably 1 to 2, units of the abovementioned resin acids with a 2- to 6-hydric alcohol, at least one free hydroxyl group being retained.

8. The process as claimed in at least one of claims 1 to 4 and 6, wherein
A is an esterification product which is obtained by esterification of 1 to 12, preferably 1 to 6, units of a naturally occurring, a hydrogenated or a disproportionated resin acid with an aminooxyalkylate comprising 1 to 5, preferably 1 to 3, units of the formula (II), at least one free hydroxyl group being retained.

9. The process as claimed in at least one of claims 1 to 8, wherein, in formula (I),
m is an integer from 5 to 30.

10. The process as claimed in at least one of claims 1 to 9, wherein the resin compound on which the radical A is based is a commercially available colophony type.

11. The process as claimed in claim 1, wherein a naturally occurring resin acid, a resin alcohol derived therefrom or a resin amine or an esterification product of the resin acids mentioned with a 2- to 6-hydric alcohol is employed as the resin compound, the esterification product still containing at least one free hydroxyl group.

12. The process as claimed in claim 1, wherein an esterification product of a resin acid mentioned in claim 1 with an aminooxyalkylate of the formula (II) mentioned in claim 1 is employed as the resin compound, the esterification product still containing at least one free hydroxyl group.

13. The process as claimed in at least one of claims 1 to 12, wherein the carboxylic acid compound A[(B)ₘ-CO-F-COOH]_{q} formed is amidated with at least one amine on which the radical Z is based.

14. The process as claimed in at least one of claims 1 to 12, wherein the carboxylic acid compound A[(B)ₘ-CO-F-COOH]_{q} formed is reacted with at least one amine on which the radical Z is based to give the amine salt.

15. The use of a compound prepared in accordance with at least one of claims 1 to 10 as a surface-active agent.

16. The use as claimed in claim 15, wherein the compound is employed as a coupling auxiliary, emulsifier, dispersing agent, preparation agent for solids dispersions, in particular coloring agent dispersions, corrosion protection and metalworking agent or as a wetting agent or dyeing auxiliary.

17. The use as claimed in claim 15, as a coupling auxiliary and preparation agent in the production of azo coloring agents for offset printing.

## Revendications (Revendications pour l'(les) Etat(s) contractant(s) suivant(s): BE, CH, DE, DK, FR, GB, IT, LI, NL)

1. Composé de formule (I)
A[(B)ₘ-Y-Z]_{q} (I)
dans laquelle
A représente le reste d'un acide résinique naturel modifié ou non modifié ou d'une amine résinique ou d'un alcool résinique qui en dérivent, ou le reste d'un produit d'estérification de 1 à 6 unités desdits acides résiniques avec un polyalcool ou d'un produit d'estérification de 1 à 12 unités desdits acides résiniques avec un amino-oxyalkylate constitué de 1 à 5 unités du composé de formule (II) 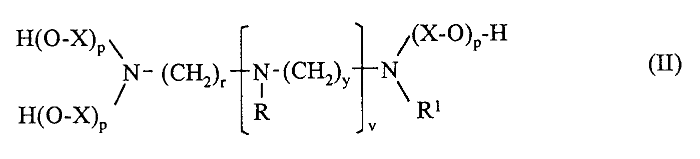
le produit d'estérification conservant au moins un groupe hydroxy libre, où
X représente un groupe ayant les formules -CH₂CH₂-, -CH₂CH(CH₃)- ou -CH(CH₃)CH₂- ou une de leurs combinaisons,
R représente un atome d'hydrogène ou le groupe -(X-O)ₚ-H,
R¹ représente le groupe -(X-O)ₚ-H ou le groupe -(X-O)ₚ-R², où R² représente un groupe divalent -OC-E-CO- qui relie, par l'intermédiaire des valences libres indiquées, deux unités du composé de formule (II) par des liaisons de type ester, et dans lequel E représente un reste aromatique divalent de 6 à 12 atomes de carbone ou un groupe alkylène linéaire, ramifié ou cycloaliphatique de 1 à 16 atomes de carbone, v représente un nombre entier de 0 à 4, et r et y sont identiques ou différents et sont chacun un nombre entier de 1 à 5;
p représente un nombre entier de 1 à 100,
B est une liaison directe lorsque A représente un produit d'estérification d'au moins un des composés résiniques précités avec au moins un reste d'un composé de formule (II),
ou un groupe de formule -(X-O)- dans lequel X a la signification donnée ci-dessus,
Y représente un groupe ayant les formules -OC-F-CO- et -OC-F-COO⁻, où F est un reste aromatique divalent de 6 à 12 atomes de carbone ou un groupe alkylène linéaire, ramifié ou cycloaliphatique de 1 à 16 atomes de carbone, et
Z représente un groupe de formule (IIIa) 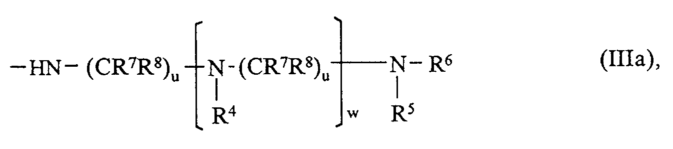 lorsque Y signifie -OC-F-CO-,
ou un cation de formule (IIIb) 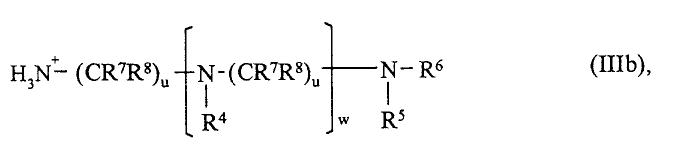 lorsque Y signifie -OC-F-COO⁻,
où R⁴, R⁵ et R⁶ représentent indépendamment les uns des autres un atome d'hydrogène ou un hydroxyalkylène de 1 à 6 atomes de carbone, R⁷ et R⁸ représentent indépendamment l'un de l'autre un hydrogène ou un méthyle, les u sont identiques ou différents et représentent un nombre entier de 1 à 14, w est un nombre entier de 0 à 25, et
m est un nombre de 1 à 100, et
q est un nombre entier de 1 à 11.

2. Composé selon la revendication 1, caractérisé en ce que, dans le groupe Z de formule (IIIa) et (IIIb),
R⁴ et R⁵ représentent chacun un atome d'hydrogène,
R⁶ représente un atome d'hydrogène ou un hydroxyalkylène de 2 à 3 atomes de carbone.

3. Composé selon la revendication 1 ou 2, caractérisé en ce que, dans le groupe Z de formule (IIIa) ou (IIIb),
R⁴, R⁵ et R⁶ représentent chacun un atome d'hydrogène,
les u sont identiques ou différents et représentent le nombre 2 ou 3 et w représente un nombre entier de 0 à 5.

4. Composé selon l'une au moins des revendications 1 à 3, caractérisé en ce que
Y représente un groupe ayant les formules -OC-F-CO- et -OC-F-COO⁻ dans lesquelles F représente un reste aromatique divalent de 6 à 8 atomes de carbone ou un groupe alkylène linéaire ou ramifié de 1 à 8 atomes de carbone, de préférence de 1 à 4 atomes de carbone.

5. Composé selon l'une au moins des revendications 1 à 4, caractérisé en ce que
B représente un groupe de formule -(X-O)-, dans laquelle X représente un groupe ayant les formules -CH₂CH₂-, -CH₂CH(CH₃)- et -CH(CH₃)CH₂- ou une de leurs combinaisons.

6. Composé selon l'une au moins des revendications 1 à 4, caractérisé en ce que
B représente une liaison directe lorsque A est un produit d'estérification préparé à partir d'au moins un des composés résiniques cités dans la revendication 1 et d'un amino-oxyalkylate contenant 1 à 5, de préférence 1 à 3 unités du composé de formule (II).

7. Composé selon au moins l'une des revendications 1 à 5, caractérisé en ce que
A représente le reste d'un acide résinique naturel, d'un acide résinique hydrogéné ou ayant subi une dismutation, d'une amine résinique ou d'un alcool résinique qui en dérivent, ou le reste d'un produit d'estérification que l'on a obtenu par estérification de 1 à 6, de préférence de 1 à 2 unités des acides résiniques précités avec un alcool ayant 2 à 6 fonctions alcool, en conservant au moins un groupe hydroxy libre.

8. Composé selon au moins l'une des revendications 1 à 4 et 6, caractérisé en ce que
A représente le reste d'un produit d'estérification que l'on a obtenu par estérification de 1 à 12, de préférence de 1 à 6 unités d'un acide résinique naturel, hydrogéné ou ayant subi une dismutation, avec un amino-oxyalkylate contenant 1 à 5, de préférence 1 à 3 unités de formule (II), en conservant au moins un groupe hydroxy libre.

9. Composé selon l'une au moins des revendications 1 à 8, caractérisé en ce que, dans la formule (I),
m représente un nombre entier de 5 à 30.

10. Composé selon l'une au moins des revendications 1 à 9, caractérisé en ce que le composé résinique dont dérive le reste A est un type de colophane courant dans le commerce.

11. Procédé de préparation du composé selon la revendication 1, caractérisé en ce que l'on effectue l'hémiestérification
a) d'acides résiniques naturels, d'alcools résiniques ou d'amines résiniques qui en dérivent, d'acides résiniques ayant subi une dismutation ou hydrogénés, ou de produits d'estérification desdits acides résiniques avec des polyalcools, les produits d'estérification conservant au moins un groupe hydroxy, oxyalkylés avec de l'oxyde d'éthylène ou de l'oxyde de propylène ou successivement avec les deux époxydes ou avec un mélange des deux époxydes, la quantité d'époxyde utilisée étant de 1 à 100 moles d'époxyde par atome d'hydrogène réactif du composé résinique utilisé, ou de produits d'oxyalkylation que l'on obtient en estérifiant lesdits acides résiniques avec des amino-oxyalkylates de formule (II),
b) avec des diacides carboxyliques ou des anhydrides de diacides carboxyliques et
c) on transforme ensuite les groupes carboxy libres du composé d'acide carboxylique formé A[(B)ₘ-CO-F-COOH]_{q} en la forme d'amide ou de sel correspondante avec au moins une diamine ou polyamine dont dérive la formule Z.

12. Procédé selon la revendication 11, caractérisé en ce que l'on utilise comme composé résinique un acide résinique naturel, un alcool résinique ou une amine résinique qui en dérivent ou un produit d'estérification desdits acides résiniques avec un alcool ayant 2 à 6 fonctions alcool, le produit d'estérification conservant au moins un groupe hydroxy libre.

13. Procédé selon la revendication 11, caractérisé en ce que l'on utilise comme composé résinique un produit d'estérification des acides résiniques cités dans la revendication 11 avec un amino-oxyalkylate ayant la formule (II) indiquée dans la revendication 1, le produit d'estérification conservant au moins un groupe hydroxy libre.

14. Procédé selon l'une au moins des revendications 11 à 13, caractérisé en ce que l'on amide le composé d'acide carboxylique A[(B)ₘ-CO-F-COOH]_{q} formé avec au moins une amine à la base du reste Z.

15. Procédé selon l'une au moins des revendications 11 à 13, caractérisé en ce que l'on transforme en sel d'amine le composé d'acide carboxylique A[(B)ₘ-CO-F-COOH]_{q} formé avec au moins une amine à la base du reste Z.

16. Utilisation d'un composé défini selon au moins l'une des revendications 1 à 10 comme agent tensioactif.

17. Utilisation selon la revendication 16, caractérisée en ce que le composé est utilisé comme auxiliaire de copulation, agent émulsionnant, agent dispersant, agent de préparation pour des dispersions de produits solides, en particulier des dispersions de colorants, agent anticorrosion et agent de traitement des métaux, ainsi que comme agent mouillant et auxiliaire de teinture.

18. Utilisation selon la revendication 16 comme auxiliaire de copulation et agent de préparation dans la préparation de colorants azoïques pour l'impression offset.

## Revendications (Revendications pour l'(les) Etat(s) contractant(s) suivant(s): ES)

1. Procédé de préparation d'un composé de formule (I)
A[(B)ₘ-Y-Z]_{q} (I)
dans laquelle
A représente le reste d'un acide résinique naturel modifié ou non modifié ou d'une amine résinique ou d'un alcool résinique qui en dérivent, ou le reste d'un produit d'estérification de 1 à 6 unités desdits acides résiniques avec un polyalcool ou d'un produit d'estérification de 1 à 12 unités desdits acides résiniques avec un amino-oxyalkylate constitué de 1 à 5 unités du composé de formule (II) 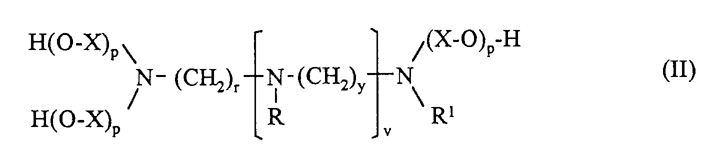
le produit d'estérification conservant au moins un groupe hydroxy libre, où
X représente un groupe ayant les formules -CH₂CH₂-, -CH₂CH(CH₃)- ou -CH(CH₃)CH₂- ou une de leurs combinaisons,
R représente un atome d'hydrogène ou le groupe -(X-O)ₚ-H,
R¹ représente le groupe -(X-O)ₚ-H ou le groupe -(X-O)ₚ-R², où R² représente un groupe divalent -OC-E-CO- qui relie, par l'intermédiaire des valences libres indiquées, deux unités du composé de formule (II) par des liaisons de type ester, et dans lequel E représente un reste aromatique divalent de 6 à 12 atomes de carbone ou un groupe alkylène linéaire, ramifié ou cycloaliphatique de 1 à 16 atomes de carbone, v représente un nombre entier de 0 à 4, et r et y sont identiques ou différents et sont chacun un nombre entier de 1 à 5;
p représente un nombre entier de 1 à 100,
B est une liaison directe lorsque A représente un produit d'estérification d'au moins un des composés résiniques précités avec au moins un reste d'un composé de formule (II),
ou un groupe de formule -(X-O)- dans lequel X a la signification donnée ci-dessus,
Y représente un groupe ayant les formules -OC-F-CO- et -OC-F-COO⁻, où F est un reste aromatique divalent de 6 à 12 atomes de carbone ou un groupe alkylène linéaire, ramifié ou cycloaliphatique de 1 à 16 atomes de carbone, et
Z représente un groupe de formule (IIIa) 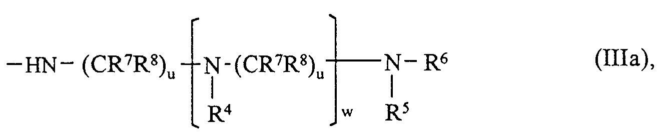 lorsque Y signifie -OC-F-CO-,
ou un cation de formule (IIIb) 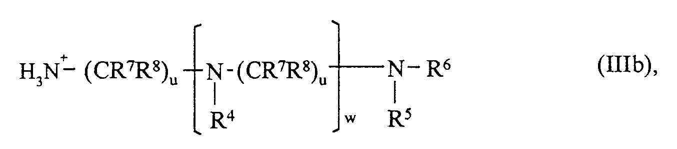 lorsque Y signifie -OC-F-COO⁻,
où R⁴, R⁵ et R⁶ représentent indépendamment les uns des autres un atome d'hydrogène ou un hydroxyalkylène de 1 à 6 atomes de carbone, R⁷ et R⁸ représentent indépendamment l'un de l'autre un hydrogène ou un méthyle, les u sont identiques ou différents et représentent un nombre entier de 1 à 14, w est un nombre entier de 0 à 25, et
m est un nombre de 1 à 100, et
q est un nombre entier de 1 à 11,
caractérisé en ce que l'on effectue l'hémiestérification
a) d'acides résiniques naturels, d'alcools résiniques ou d'amines résiniques qui en dérivent, d'acides résiniques ayant subi une dismutation ou hydrogénés, ou de produits d'estérification desdits acides résiniques avec des polyalcools, les produits d'estérification conservant au moins un groupe hydroxy, oxyalkylés avec de l'oxyde d'éthylène ou de l'oxyde de propylène ou successivement avec les deux époxydes ou avec un mélange des deux époxydes, la quantité d'époxyde utilisée étant de 1 à 100 moles d'époxyde par atome d'hydrogène réactif du composé résinique utilisé, ou de produits d'oxyalkylation que l'on obtient en estérifiant lesdits acides résiniques avec des amino-oxyalkylates de formule (II),
b) avec des diacides carboxyliques ou des anhydrides de diacides carboxyliques et
c) on transforme ensuite les groupes carboxy libres du composé d'acide carboxylique formé A[(B)ₘ-CO-F-COOH]_{q} en la forme d'amide ou de sel correspondante avec au moins une diamine ou polyamine dont dérive la formule Z.

2. Procédé selon la revendication 1, caractérisé en ce que, dans le groupe Z de formule (IIIa) et (IIIb),
R⁴ et R⁵ représentent chacun un atome d'hydrogène,
R⁶ représente un atome d'hydrogène ou un hydroxyalkylène de 2 à 3 atomes de carbone.

3. Procédé selon la revendication 1 ou 2, caractérisé en ce que, dans le groupe Z de formule (IIIa) ou (IIIb),
R⁴, R⁵ et R⁶ représentent chacun un atome d'hydrogène,
les u sont identiques ou différents et représentent le nombre 2 ou 3 et
w représente un nombre entier de 0 à 5.

4. Procédé selon l'une au moins des revendications 1 à 3, caractérisé en ce que
Y représente un groupe ayant les formules -OC-F-CO- et -OC-F-COO⁻ dans lesquelles F représente un reste aromatique divalent de 6 à 8 atomes de carbone ou un groupe alkylène linéaire ou ramifié de 1 à 8 atomes de carbone, de préférence de 1 à 4 atomes de carbone.

5. Procédé selon l'une au moins des revendications 1 à 4, caractérisé en ce que
B représente un groupe de formule -(X-O)-, dans laquelle X représente un groupe ayant les formules -CH₂CH₂-, -CH₂CH(CH₃)- ou -CH(CH₃)CH₂- ou une de leurs combinaisons.

6. Procédé selon l'une au moins des revendications 1 à 4, caractérisé en ce que
B représente une liaison directe lorsque A est un produit d'estérification préparé à partir d'au moins un des composés résiniques cités dans la revendication 1 et d'un amino-oxyalkylate contenant 1 à 5, de préférence 1 à 3 unités du composé de formule (II).

7. Procédé selon au moins l'une des revendications 1 à 5, caractérisé en ce que
A représente le reste d'un acide résinique naturel, d'un acide résinique hydrogéné ou ayant subi une dismutation, d'une amine résinique ou d'un alcool résinique qui en dérivent, ou le reste d'un produit d'estérification que l'on a obtenu par estérification de 1 à 6, de préférence de 1 à 2 unités des acides résiniques précités avec un alcool ayant 2 à 6 fonctions alcool, en conservant au moins un groupe hydroxy libre.

8. Procédé selon au moins l'une des revendications 1 à 4 et 6, caractérisé en ce que
A représente le reste d'un produit d'estérification que l'on a obtenu par estérification de 1 à 12, de préférence de 1 à 6 unités d'un acide résinique naturel, hydrogéné ou ayant subi une dismutation, avec un amino-oxyalkylate contenant 1 à 5, de préférence 1 à 3 unités de formule (II), en conservant au moins un groupe hydroxy libre.

9. Procédé selon l'une au moins des revendications 1 à 8, caractérisé en ce que, dans la formule (I),
m représente un nombre entier de 5 à 30.

10. Procédé selon l'une au moins des revendications 1 à 9, caractérisé en ce que le composé résinique dont dérive le reste A est un type de colophane courant dans le commerce.

11. Procédé selon la revendication 1, caractérisé en ce que l'on utilise comme composé résinique un acide résinique naturel, un alcool résinique ou une amine résinique qui en dérivent ou un produit d'estérification desdits acides résiniques avec un alcool ayant 2 à 6 fonctions alcool, le produit d'estérification conservant au moins un groupe hydroxy libre.

12. Procédé selon la revendication 1, caractérisé en ce que l'on utilise comme composé résinique un produit d'estérification des acides résiniques cités dans la revendication 11 avec un amino-oxyalkylate ayant la formule (II) indiquée dans la revendication 1, le produit d'estérification conservant au moins un groupe hydroxy libre.

13. Procédé selon l'une au moins des revendications 1 à 12, caractérisé en ce que l'on amide le composé d'acide carboxylique formé A[(B)ₘ-CO-F-COOH]_{q} avec au moins une amine à la base du reste Z.

14. Procédé selon l'une au moins des revendications 1 à 12, caractérisé en ce que l'on transforme en sel d'amine le composé d'acide carboxylique A[(B)ₘ-CO-F-COOH]_{q} formé avec au moins une amine à la base du reste Z.

15. Utilisation d'un composé préparé selon au moins l'une des revendications 1 à 10 comme agent tensioactif.

16. Utilisation selon la revendication 15, caractérisée en ce que le composé est utilisé comme auxiliaire de copulation, agent émulsionnant, agent dispersant, agent de préparation pour des dispersions de produits solides, en particulier des dispersions de colorants, agent anticorrosion et agent de traitement des métaux, ainsi que comme agent mouillant et auxiliaire de teinture.

17. Utilisation selon la revendication 15 comme auxiliaire de copulation et agent de préparation dans la préparation de colorants azoïques pour l'impression offset.
